(19) Europäisches Patentamt
European Patent Office
Office européen des brevets

(11) Veröffentlichungsnummer: **0 679 644 A1**

(12) # EUROPÄISCHE PATENTANMELDUNG

(21) Anmeldenummer: **95105723.1**

(22) Anmeldetag: **18.04.95**

(51) Int. Cl.[6]: **C07D 231/38**, C07D 403/04, A01N 47/38

(30) Priorität: **29.04.94 DE 4414974**

(43) Veröffentlichungstag der Anmeldung: **02.11.95 Patentblatt 95/44**

(84) Benannte Vertragsstaaten: **BE CH DE ES FR GB IT LI NL PT**

(71) Anmelder: **BAYER AG**
**D-51368 Leverkusen (DE)**

(72) Erfinder: **Fuchs, Rainer, Dr.**
**Am Rohm 107**
**D-42113 Wuppertal (DE)**
Erfinder: **Erdelen, Christoph, Dr.**
**Unterbüscherhof 15**
**D-42799 Leichlingen (DE)**

(54) **Substituierte Pyrazolinderivate.**

(57) Die Erfindung betrifft neue substituierte Pyrazolinderivate, Verfahren zu ihrer Herstellung und ihre Verwendung als Schädlingsbekämpfungsmittel, insbesondere als Insektizide und Akarizide. Die neuen Verbindungen besitzen die Formel

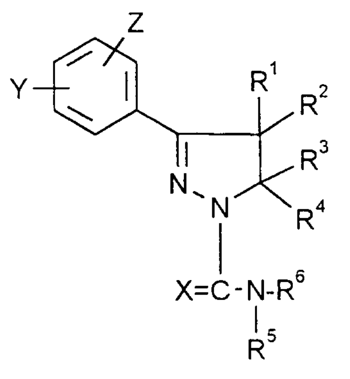

(I)

in welcher

| | |
|---|---|
| $R^1$ | für einen ungesättigten fünfgliedrigen, 1 bis 4 Stickstoffatome enthaltenden, gegebenenfalls substituierten und gegebenenfalls benzokondensierten Heterocyclus steht, |
| $R^2$ | für Wasserstoff, Alkyl, gegebenenfalls substituiertes Cycloalkyl, Halogenalkyl, Halogenalkylthio oder Alkoxycarbonyl steht, |
| $R^3$ | für Wasserstoff oder Alkyl steht, |
| $R^4$ | für Wasserstoff oder Alkyl steht, |
| $R^5$ | für Cyano, für jeweils gegebenenfalls substituiertes Alkyl, Cycloalkyl, Alkenyl oder Alkinyl, für Formyl oder für jeweils gegebenenfalls substituiertes Alkylcarbonyl oder Alkoxycarbonyl steht, |
| X | für Sauerstoff oder Schwefel steht und |
| $R^6$, Y und Z | die im Anmeldungstext angegebene Bedeutung haben. |

EP 0 679 644 A1

Die Erfindung betrifft neue substituierte Pyrazolinderivate, Verfahren zu ihrer Herstellung und ihre Verwendung als Schädlingsbekämpfungsmittel.

Es ist bekannt, daß bestimmte substituierte Pyrazolinderivate, wie 3-(4'-Chlorphenyl)-4-(1"H-4"-chlorpyrazol-1"-yl)-4,5-dihydro-1-pyrazolcarbonsäure-(4-trifluormethoxy)-anilid und 3-(4'-chlorphenyl)-4-(1"H-4"-chlorpyrazol-1"-yl)-4,5-dihydro-1-pyrazolcarbonsäure-(4-trifluormethyl)-anilid, Wirksamkeit gegen tierische Schädlinge besitzen (vgl. EP-A-0 438 690).

Die Wirkungshöhe bzw. Wirkungsdauer dieser vorbekannten Verbindungen ist jedoch, insbesondere gegen bestimmte Organismen oder bei niedrigen Anwendungskonzentrationen,nicht in allen Anwendungsgebieten völlig zufriedenstellend.

Es wurden neue substituierte Pyrazolinderivate der Formel (I)

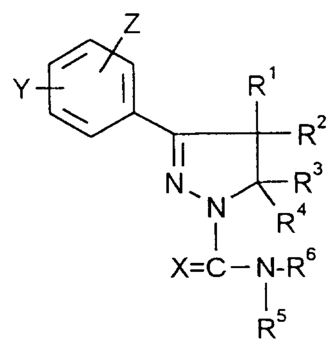

(I)

gefunden, in welcher

$R^1$ für einen ungesättigten fünfgliedrigen, 1 bis 4 Stickstoffatome enthaltenden, gegebenenfalls substituierten und gegebenenfalls benzokondensierten Heterocyclus steht,

$R^2$ für Wasserstoff, Alkyl, gegebenenfalls substituiertes Cycloalkyl, Halogenalkyl, Halogenalkylthio, Alkoxycarbonyl oder Trialkylsilyl steht,

$R^3$ für Wasserstoff oder Alkyl steht,

$R^4$ für Wasserstoff oder Alkyl steht,

$R^5$ für Cyano, für jeweils gegebenenfalls substituiertes Alkyl, Cycloalkyl, Alkenyl oder Alkinyl, für Formyl, für jeweils gegebenenfalls substituiertes Alkylcarbonyl oder Alkoxycarbonyl steht,

$R^6$ für gegebenenfalls substituiertes Alkyl, gegebenenfalls substituiertes Cycloalkyl oder für den Rest

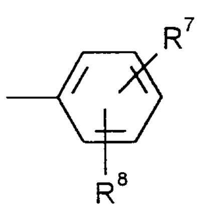

steht,

wobei $R^7$ und $R^8$ gleich oder verschieden sein können und für Wasserstoff, Halogen, Alkyl, Nitro, Cyano, Halogenalkyl, Alkoxy, Halogenalkoxy, Alkylthio, Halogenalkylthio, jeweils gegebenenfalls substituiertes Phenoxy oder Phenylthio, gegebenenfalls substituiertes Mono- oder Dialkylamino, gegebenenfalls substituiertes Cycloalkyl, Alkoxycarbonyl, Alkenyl, Alkinyl, Alkylthionyl, Alkylsulfonyl, Halogenalkylthionyl, Halogenalkylsulfonyl, Halogenalkoxycarbonyl oder wobei $R^7$ und $R^8$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z unabhängig voneinander für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, jeweils gegebenenfalls substituiertes Aryloxy oder Arylthio, Alkenyl, Alkinyl, Alkylthionyl, Alkylsulfonyl, Halogenalkylthionyl, Halogenalkylsulfonyl, Amino, gegebenenfalls substituiertes Mono- oder Dialkylamino, Nitro, Cyano stehen oder Y und Z zusammen für gegebenenfalls durch Halogen substituiertes 3,4-Methylendioxy oder 3,4-Ethylendioxy stehen.

Die Verbindungen der Formel (I) können auch, in Abhängigkeit von der Art der Substituenten, als geometrische und/oder optische Isomere oder Isomerengemische unterschiedlicher Zusammensetzung vorliegen. Die Erfindung betrifft sowohl die reinen Isomeren als auch die Isomerengemische.

Weiterhin wurde gefunden, daß man die neuen substituierten Pyrazolinderivate der Formel (I) erhält, wenn man

a) substituierte Pyrazolinderivate der Formel (II)

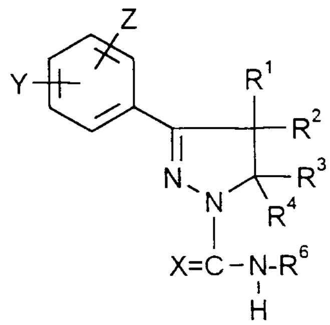

(II)

in welcher

$R^1$, $R^2$, $R^3$, $R^4$, $R^6$, X, Y und Z die oben angegebene Bedeutung haben, mit Verbindungen der Formel (III)

$R^5$-A (III)

in welcher

$R^5$ die oben angegebene Bedeutung hat und

A für eine geeignete Abgangsgruppe steht

in Gegenwart einer Base und gegebenenfalls in Gegenwart eines Verdünnungsmittels umsetzt oder

b) substituierte Pyrazoline der Formel (IV)

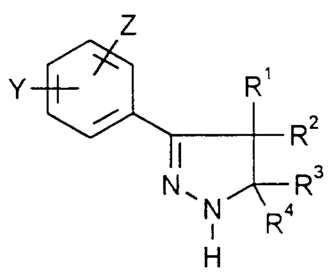

(IV)

in welcher

$R^1$, $R^2$, $R^3$, $R^4$, Y und Z die oben angegebene Bedeutung haben,

mit Verbindungen der Formel (V)

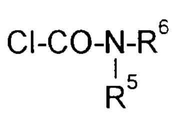

(V)

in welcher

$R^5$ und $R^6$ die oben angegebene Bedeutung haben

gegebenenfalls in Gegenwart einer Base und gegebenenfalls in Gegenwart eines Verdünnungsmittels umsetzt.

Schließlich wurde gefunden, daß die neuen Pyrazolinderivate der Formel (I) eine sehr gute Wirksamkeit gegen Schädlinge und insbesondere eine sehr gute insektizide und akarizide Wirksamkeit besitzen.

Überraschenderweise zeigen die erfindungsgemäßen substituierten Pyrazolinderivate eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten Pyrazolinderivate sind durch die Formel (I) allgemein definiert.

Bevorzugte Substituenten bzw. Bereiche der in den oben und nachstehend erwähnten Formeln aufgeführten Reste werden im folgenden erläutert:

$R^1$ steht bevorzugt für einen gegebenenfalls einfach oder mehrfach gleich oder verschieden durch Halogen, Alkyl($C_1$-$C_6$), CN, $NO_2$, Alkoxy($C_1$-$C_6$)carbonyl, Alkyl($C_1$-$C_4$)thio, Alkoxy-($C_1$-$C_6$), Halogenalkyl($C_1$-$C_4$), Halogenalkyl($C_1$-$C_4$)thio, Halogenalkoxy($C_1$-$C_4$), Amino, Mono- oder Dialkyl($C_1$-$C_4$)amino oder Halogenalkoxy($C_1$-$C_6$)carbonyl substituierten 1H-Pyrrolyl-(1)-, 1H-Pyrazolyl-(1)-, 1H-Imidazolyl-(1)-, 2H-1,2,3,-Triazolyl-(2)-, 1H-1,2,3-Triazolyl-(1)-, 1H-1,2,4,-Triazolyl-(1)-, 4H-1,2,4-Triazolyl-(4)-, 2H-Tetrazolyl-(2)- oder 1H-Tetrazolyl-(1)-Rest.

$R^2$ steht bevorzugt für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$), Halogenalkyl-($C_1$-$C_4$)thio, Alkoxy($C_1$-$C_6$)carbonyl oder Trialkyl($C_1$-$C_6$)silyl.

$R^3$ steht bevorzugt für Wasserstoff oder Alkyl($C_1$-$C_6$).

$R^4$ steht bevorzugt für Wasserstoff oder Alkyl($C_1$-$C_6$).

$R^5$ steht bevorzugt für Cyano, für gegebenenfalls durch Halogen, Alkoxy($C_1$-$C_6$) oder einen der Reste

$CO_2R^7$ oder $NR^8R^9$

substituiertes Alkyl($C_1$-$C_{10}$),

wobei

$R^7$ für gegebenenfalls durch Halogen substituiertes Alkyl($C_1$-$C_{10}$) steht,

$R^8$ und $R^9$ gleich oder verschieden sind und für Alkyl($C_1$-$C_{10}$) stehen oder

$R^8$ und $R^9$ gemeinsam mit dem Stickstoffatom, an das sie gebunden sind einen 3- bis 8-gliedrigen Ring bilden, der gegebenenfalls ein weiteres Heteroatom enthält,

für gegebenenfalls durch Halogen oder Halogenalkyl($C_1$-$C_6$) substituiertes Cycloalkyl($C_3$-$C_8$), für jeweils gegebenenfalls durch Halogen oder Alkoxy($C_1$-$C_6$) substituiertes Alkenyl-($C_2$-$C_8$) oder Alkinyl($C_2$-$C_8$), für Formyl, für gegebenenfalls durch Halogen substituiertes Alkyl($C_1$-$C_{10}$)carbonyl oder für gegebenenfalls durch Halogen substituiertes Alkoxy($C_1$-$C_{10}$)carbonyl.

$R^6$ steht bevorzugt für gegebenenfalls durch Halogen oder Halogenalkoxy($C_1$-$C_4$) substituiertes Alkyl($C_1$-$C_6$),

für gegebenenfalls durch Halogen, Halogenalkyl($C_1$-$C_4$), Halogenalkoxy($C_1$-$C_4$) substituiertes Cycloalkyl($C_3$-$C_7$) oder für den Rest

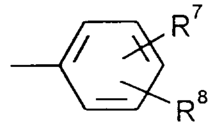

wobei $R^7$ und $R^8$ gleich oder verschieden sein können und für Wasserstoff, Halogen, Alkyl($C_1$-$C_6$), Nitro, Cyano, Halogenalkyl($C_1$-$C_6$), Alkoxy($C_1$-$C_6$), Halogenalkoxy($C_1$-$C_6$), Alkyl($C_1$-$C_6$)thio, Halogenalkyl($C_1$-$C_6$)thio, jeweils gegebenenfalls durch Halogen, Halogenalkyl($C_1$-$C_6$), Alkoxy($C_1$-$C_6$), Alkyl($C_1$-$C_6$) substituiertes Phenoxy oder Phenylthio, gegebenenfalls durch Halogen, Alkoxy($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy-($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$), Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)carbonyl stehen oder wobei $R^7$ und $R^8$ zusammen für einen der folgenden bivalenten Reste stehen:

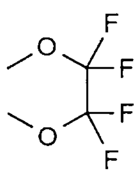 , 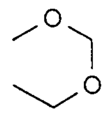 , 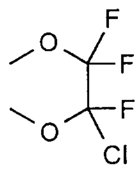 , 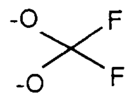 .

X steht bevorzugt für Sauerstoff oder Schwefel.

Y und Z stehen unabhängig voneinander bevorzugt für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_6$), Halogenalkyl-($C_1$-$C_6$)thio, Alkoxy($C_1$-$C_4$)-carbonyl, Halogenalkoxy($C_1$-$C_4$)carbonyl, jeweils gegebenenfalls durch Halogen, Alkyl($C_1$-$C_4$), Alkoxy($C_1$-$C_4$), Halogenalkyl($C_1$-$C_4$) substituiertes Phenoxy oder Phenylthio, Alkenyl($C_2$-$C_6$), Alkinyl($C_2$-$C_6$), Alkyl($C_1$-$C_4$)-thionyl, Alkyl($C_1$-$C_4$)-sulfonyl, Halogenalkyl($C_1$-$C_4$)thionyl, Halogenalkyl($C_1$-$C_4$)sulfonyl, gegebenenfalls durch Halogen, Alkoxy($C_1$-$C_4$) substituiertes Mono- oder Dialkyl($C_1$-$C_6$)amino, Nitro oder Cyano oder

Y und Z stehen gemeinsam bevorzugt für gegebenenfalls durch Fluor und/oder Chlor substituiertes 3,4-Methylendioxy oder 3,4-Ethylendioxy.

$R^1$ steht besonders bevorzugt für einen gegebenenfalls ein- oder zweifach gleich oder verschieden durch Fluor, Chlor, Brom, Jod, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl($C_1$-$C_3$)thio, Amino oder Dialkyl($C_1$-$C_3$)amino oder Halogenalkoxy($C_1$-$C_4$)carbonyl substituierten 1-H-Pyrrolyl-(1)-, 1H-Pyrazolyl-(1)-, 1H-Imidazolyl-(1)-, 2H-1,2,3-Triazolyl-(2)-, 1H-1,2,3-Triazolyl-(1)-, 1H,1,2,4-Triazolyl-(1)-, 4H-1,2,4-Triazolyl-(4)-, 2H-Tetrazolyl-(2) oder 1H-Tetrazolyl-(1)-Rest.

$R^2$ steht besonders bevorzugt für Wasserstoff Alkyl($C_1$-$C_4$), gegebenenfalls durch Fluor, Chlor, Brom oder Halogenalkyl($C_1$-$C_3$) substituiertes Cycloalkyl ($C_3$-$C_6$), Halogenalkyl($C_1$-$C_3$), Halogenalkyl($C_1$-$C_3$)thio, Alkoxy($C_1$-$C_4$)carbonyl oder Trialkyl($C_1$-$C_4$)silyl.

$R^3$ steht besonders bevorzugt für Wasserstoff oder Alkyl($C_1$-$C_4$).

$R^4$ steht besonders bevorzugt für Wasserstoff oder Alkyl($C_1$-$C_4$).

$R^5$ steht besonders bevorzugt für Cyano, für gegebenenfalls durch Fluor, Chlor, Brom, Alkoxy($C_1$-$C_4$) oder einen der Reste

$CO_2R^7$ oder $NR^8R^9$

substituiertes Alkyl($C_1$-$C_8$),

wobei

$R^7$ für gegebenenfalls durch Fluor, Chlor und/oder Brom substituiertes Alkyl($C_1$-$C_8$) steht,

$R^8$ und $R^9$ gleich oder verschieden sind und für Alkyl($C_1$-$C_8$) stehen oder gemeinsam mit dem Stickstoffatom, an das sie gebunden sind einen 3- bis 7-gliedrigen Ring bilden, der gegebenenfalls ein Sauerstoffatom enthält,

für gegebenenfalls durch Fluor, Chlor, Brom oder Halogenalkyl($C_1$-$C_4$) substituiertes Cycloalkyl($C_3$-$C_6$), für jeweils gegebenenfalls durch Fluor, Chlor, Brom oder Alkoxy($C_1$-$C_4$) substituiertes Alkenyl($C_3$-$C_7$) oder Alkinyl($C_3$-$C_7$), für Formyl, für jeweils gegebenenfalls durch Fluor, Chlor und/oder Brom substituiertes Alkyl($C_1$-$C_8$)carbonyl oder Alkoxy($C_1$-$C_8$)-carbonyl,

$R^6$ steht besonders bevorzugt für gegebenenfalls durch Fluor, Chlor, Brom oder Halogenalkoxy($C_1$-$C_3$) substituiertes Alkyl($C_1$-$C_6$), für gegebenenfalls durch Fluor, Chlor, Brom, Halogenalkyl($C_1$-$C_3$) oder Halogenalkoxy($C_1$-$C_3$) substituiertes Cycloalkyl($C_3$-$C_6$) oder für den Rest

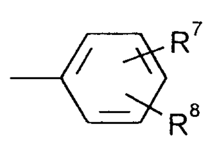

wobei $R^7$ und $R^8$ gleich oder verschieden sein können und für Wasserstoff, Fluor, Chlor, Brom, Iod, Alkyl($C_1$-$C_4$), Nitro, Cyano, Halogenalkyl($C_1$-$C_3$), Alkoxy($C_1$-$C_4$), Halogenalkoxy-

$(C_1-C_3)$ Alkyl$(C_1-C_3)$thio, Halogenalkyl$(C_1-C_3)$thio, gegebenenfalls durch Fluor, Chlor, Brom, Halogenalkyl$(C_1-C_3)$, Alkoxy$(C_1-C_3)$ oder Alkyl$(C_1-C_3)$ substituiertes Phenoxy, gegebenenfalls durch Fluor, Chlor, Brom oder Alkoxy$(C_1-C_3)$ substituiertes Mono- oder Dialkylamino mit jeweils 1 bis 4 Kohlenstoffatomen im Alkylrest, gegebenenfalls durch Alkyl$(C_1-C_3)$, Alkoxy$(C_1-C_3)$, Fluor, Chlor, Brom, Alkyl$(C_1-C_3)$thio substituiertes Cycloalkyl$(C_3-C_6)$ stehen oder wobei

$R^7$ und $R^8$ zusammen für einen der folgenden bivalenten Reste stehen:

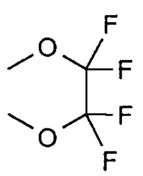 , 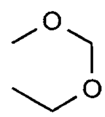 , 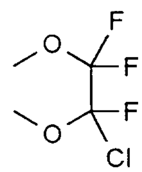 , 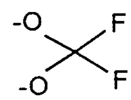 .

X steht besonders bevorzugt für Sauerstoff oder Schwefel.

Y und Z stehen unabhängig voneinander besonders bevorzugt für Wasserstoff, Alkyl$(C_1-C_4)$, Fluor, Chlor, Brom, Iod, Halogenalkyl$(C_1-C_4)$, Alkoxy$(C_1-C_4)$, Alkyl$(C_1-C_4)$thio, Halogenalkoxy$(C_1-C_3)$, Halogenalkyl$(C_1-C_3)$thio, Alkoxy$(C_1-C_3)$carbonyl, gegebenenfalls durch Fluor, Chlor, Brom, Alkyl$(C_1-C_3)$, Alkoxy$(C_1-C_3)$ oder Halogenalkyl$(C_1-C_3)$ substituiertes Phenoxy oder Phenylthio, Halogenalkoxy$(C_1-C_3)$carbonyl, Alkenyl$(C_2-C_4)$, Alkinyl$(C_2-C_4)$, Alkyl$(C_1-C_3)$-thionyl, Alkyl$(C_1-C_3)$sulfonyl, Halogenalkyl$(C_1-C_3)$sulfonyl, gegebenenfalls durch Halogen, Alkoxy$(C_1-C_3)$ substituiertes Mono- oder Dialkyl$(C_1-C_3)$amino, Nitro oder Cyano oder

Y und Z stehen gemeinsam besonders bevorzugt für gegebenenfalls durch Fluor und/oder Chlor substituiertes 3,4-Methylendioxy oder 3,4-Ethylendioxy.

$R^1$ steht ganz besonders bevorzugt für einen gegebenenfalls ein- oder zweifach gleich oder verschieden durch Chlor, Brom, Jod, Alkyl$(C_1-C_3)$, CN, $NO_2$, Alkoxy$(C_1-C_3)$carbonyl, Alkyl-$(C_1-C_2)$thio, Alkoxy$(C_1-C_3)$, Halogenalkyl$(C_1-C_3)$, Halogenalkyl$(C_1-C_2)$thio, Dialkyl$(C_1-C_2)$-amino oder Halogenalkoxy$(C_1-C_3)$carbonyl substituierten 1H-Pyrrolyl-(1)-, 1H-Pyrazolyl-(1)-, 1H-Imidazolyl-(1)-, 2H-1,2,3-Triazolyl-(2)-, 1H-1,2,3-Triazolyl-(1)-, 1H-1,2,4-Triazolyl-(1)-, 4H-1,2,4-Triazolyl-(4)-, 2H-Tetrazolyl-(2) oder 1H-Tetrazolyl-(1)-Rest.

$R^2$ steht ganz besonders bevorzugt für Wasserstoff, Alkyl$(C_1-C_3)$, Halogenalkyl$(C_1-C_2)$, Alkoxy$(C_1-C_3)$carbonyl oder Trialkyl$(C_1-C_3)$silyl.

$R^3$ steht ganz besonders bevorzugt für Wasserstoff, Methyl, Ethyl oder Propyl.

$R^4$ steht ganz besonders bevorzugt für Wasserstoff, Methyl, Ethyl oder Propyl.

$R^5$ steht ganz besonders bevorzugt für Cyano, für gegebenenfalls durch Fluor, Chlor, Alkoxy-$(C_1-C_3)$ oder einen der Reste

$CO_2R^7$ oder $NR^8R^9$

substituiertes Alkyl$(C_1-C_6)$,

wobei

$R^7$ für gegebenenfalls durch Fluor und/oder Chlor substituiertes Alkyl$(C_1-C_6)$ steht,

$R^8$ und $R^9$ gleich oder verschieden sind und für Alkyl$(C_1-C_6)$ stehen oder gemeinsam mit dem Stickstoffatom, an das sie gebunden sind einen 5- oder 6-gliedrigen Ring bilden, der gegebenenfalls ein Sauerstoffatom enthält,

für gegebenenfalls durch Fluor, Chlor oder Halogenalkyl$(C_1-C_3)$ substituiertes Cycloalkyl mit 3, 5 oder 6 C-Atomen, für jeweils gegebenenfalls durch Fluor, Chlor oder Alkoxy$(C_1-C_3)$ substituiertes Alkenyl$(C_3-C_6)$ oder Alkinyl$(C_3-C_6)$, für Formyl, für jeweils gegebenenfalls durch Fluor und/oder Chlor substituiertes Alkyl$(C_1-C_6)$carbonyl oder Alkoxy$(C_1-C_6)$-carbonyl.

$R^6$ steht ganz besonders bevorzugt für gegebenenfalls durch Fluor, Chlor, Halogenalkyl$(C_1-C_3)$ oder Halogenalkoxy$(C_1-C_2)$ substituiertes Cycloalkyl$(C_3-C_6)$ oder für den Rest

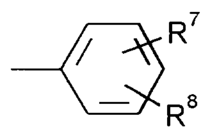

wobei $R^7$ und $R^8$ gleich oder verschieden sein können und für Wasserstoff, Fluor, Chlor, Brom, Alkyl($C_1$-$C_3$), Nitro, Cyano, Halogenalkyl($C_1$-$C_3$), Alkoxy($C_1$-$C_3$), Halogenalkoxy($C_1$-$C_2$), Alkyl($C_1$-$C_2$)thio, Halogenalkyl($C_1$-$C_2$)thio, gegebenenfalls durch Fluor, Chlor, Brom, Halogenalkyl($C_1$-$C_2$), Alkoxy($C_1$-$C_2$) oder Alkyl($C_1$-$C_2$) substituiertes Phenoxy, gegebenenfalls durch Fluor, Chlor oder Alkoxy($C_1$-$C_3$) substituiertes Mono- oder Dialkylamino mit jeweils 1 bis 3 Kohlenstoffatomen im Alkylrest, gegebenenfalls durch Alkyl($C_1$-$C_2$), Alkoxy-($C_1$-$C_2$), Fluor, Chlor oder Alkyl($C_1$-$C_2$)thio substituiertes Cycloalkyl mit 3,5 oder 6 C-Atomen stehen oder

$R^7$ und $R^8$ zusammen für eine der folgenden bivalenten Reste stehen:

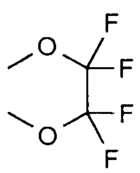 , 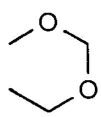 , 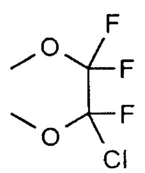 , 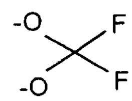

X steht ganz besonders bevorzugt für Sauerstoff oder Schwefel.

Y und Z stellen unabhängig voneinander ganz besonders bevorzugt für Wasserstoff, Alkyl($C_1$-$C_3$), Fluor, Chlor, Brom, Halogenalkyl($C_1$-$C_3$), Alkoxy($C_1$-$C_3$), Alkyl($C_1$-$C_3$)thio, Halogenalkoxy-($C_1$-$C_2$), Halogenalkyl($C_1$-$C_2$)thio, Alkoxy($C_1$-$C_2$)carbonyl, gegebenenfalls durch Fluor, Chlor, Brom, Alkyl($C_1$-$C_3$), Alkoxy($C_1$-$C_3$) oder Halogenalkyl($C_1$-$C_3$) substituiertes Phenoxy, Halogenalkoxy($C_1$-$C_2$)carbonyl, Alkenyl($C_2$-$C_4$), Alkyl($C_1$-$C_3$)thionyl, Alkyl($C_1$-$C_3$)-sulfonyl, Halogenalkyl($C_1$-$C_3$)sulfonyl, gegebenenfalls durch Fluor, Chlor, Alkoxy($C_1$-$C_2$), substituiertes Mono- oder Dialkyl($C_1$-$C_3$)amino, Nitro oder Cyano oder

Y und Z stehen gemeinsam ganz besonders bevorzugt für gegebenenfalls durch Fluor und/oder Chlor substituiertes 3,4-Methylendioxy oder 3,4-Ethylendioxy.

Die Substituentenbedeutung Halogenalkyl in den Resten Halogenalkyl, Halogenalkoxy, Halogenalkylthio, Halogenalkylthionyl und Halogenalkylsulfonyl enthält bevorzugt 1 bis 9, besonders bevorzugt 1 bis 7 und ganz besonders bevorzugt 1 bis 5 gleiche oder verschiedene Halogenatome aus der Reihe Fluor, Chlor und Brom, vorzugsweise Fluor und/oder Chlor.

Die oben aufgeführten Kohlenwasserstoffreste können auch in Verbindung mit Heteroatomen wie in Alkoxy oder Alkylsulfonyl, soweit möglich jeweils geradkettiges oder verzweigt sein.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der allgemeinen Formel (I), in welchen sie eine Kombination der vorstehend als bevorzugt aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I), in welchen eine Kombination der vorstehend als ganz besonders bevorzugt aufgeführten Bedeutungen vorliegt.

Im einzelnen seien außer den bei den Herstellungsbeispielen genannten Verbindungen die folgenden substituierten Pyrazolinderivate der Formel (I) genannt:

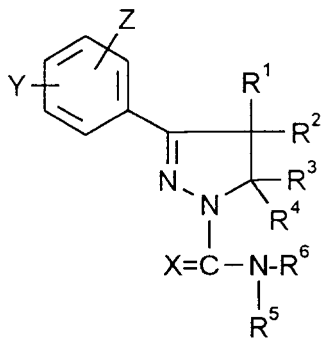

(I)

Tabelle I

| Y | Z | X | $R^1$ | $R^2$ | $R^3$ | $R^4$ | $R^5$ | $R^6$ |
|---|---|---|---|---|---|---|---|---|
| H | H | O | 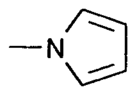 | H | H | H | $CH_3$ | 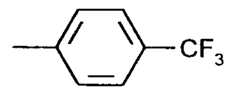 |
| H | H | O | 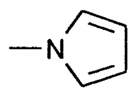 | H | H | H | $CH_3$ | 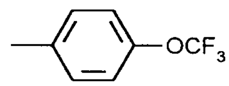 |
| 4-$CF_3$ | H | O | 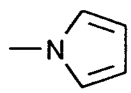 | H | H | H | $CH_3$ | 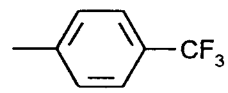 |
| 4-$CF_3$ | H | O | 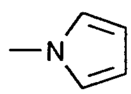 | H | H | H | $CH_3$ | 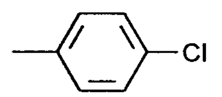 |
| 4-$CF_3$ | H | O | 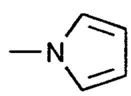 | H | H | H | $CH_3$ | 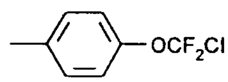 |
| 4-Br | H | O | 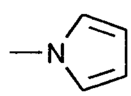 | H | H | H | $CH_3$ | 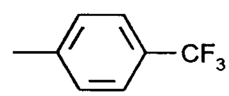 |
| 3-$CF_3$ | H | O | 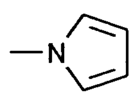 | H | H | H | $CH_3$ | 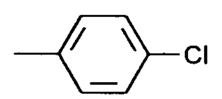 |
| 3-$CF_3$ | H | O | 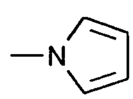 | H | H | H | $CH_3$ | 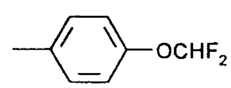 |
| 4-F | H | O | 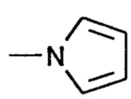 | H | H | H | $CH_3$ | 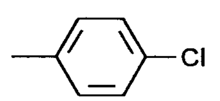 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 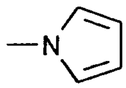 | H | H | H | $CH_3$ | 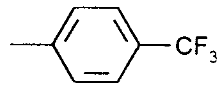 |
| 4-F | H | O | 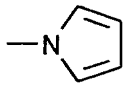 | H | H | H | $CH_3$ | 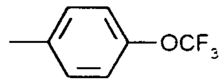 |
| 4-F | H | O | 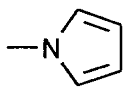 | H | H | H | $CH_3$ | 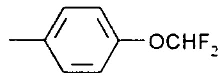 |
| 4-F | H | O | 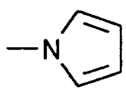 | H | H | H | $CH_3$ | 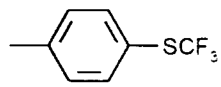 |
| 4-F | H | O | 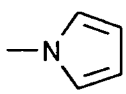 | H | H | H | $CH_3$ | 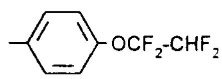 |
| 4-F | H | O | 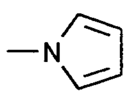 | H | H | H | $CH_3$ | 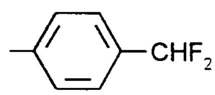 |
| 4-F | H | O | 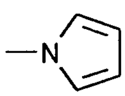 | H | H | H | $CH_3$ | 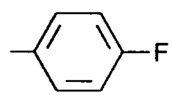 |
| 4-F | H | O | 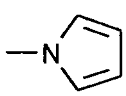 | H | H | H | $CH_3$ | 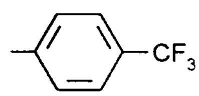 |
| 4-$OCHF_2$ | H | O | 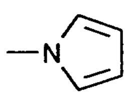 | H | H | H | $CH_3$ | 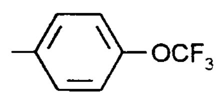 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 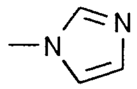 | H | H | H | $CH_3$ | 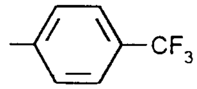 |
| 4-F | H | O | 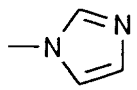 | H | H | H | $CH_3$ | 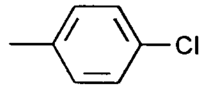 |
| 4-F | H | O | 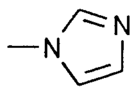 | H | H | H | $CH_3$ | 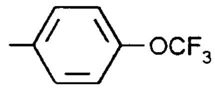 |
| 4-F | H | O | 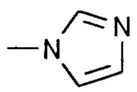 | H | H | H | $CH_3$ | 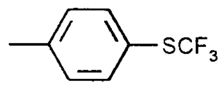 |
| 4-F | H | O | 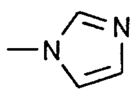 | H | H | H | $CH_3$ | 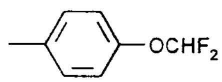 |
| 4-Cl | H | O | 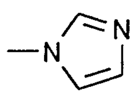 | H | H | H | $CH_3$ | 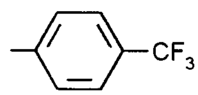 |
| 4-Cl | H | O | 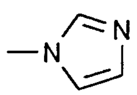 | H | H | H | $CH_3$ | 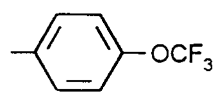 |
| 4-Cl | H | O | 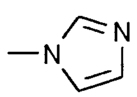 | H | H | H | $CH_3$ | 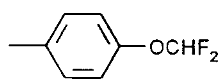 |
| 4-Cl | H | O | 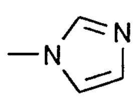 | H | H | H | $CH_3$ | 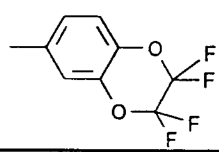 |

<u>Tabelle 1</u> - Fortsetzung

| Y | Z | X | $R^1$ | $R^2$ | $R^3$ | $R^4$ | $R^5$ | $R^6$ |
|---|---|---|---|---|---|---|---|---|
| 4-$OCHF_2$ | H | O | 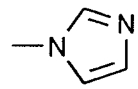 | H | H | H | $CH_3$ | 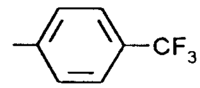 |
| 4-Cl | H | O | 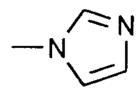 | H | H | H | $CH_3$ | 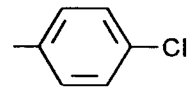 |
| 4-$OCHF_2$ | H | O | 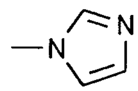 | H | H | H | $CH_3$ | 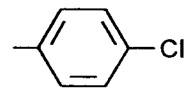 |
| 4-Cl | H | O | 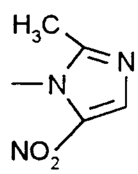 | H | H | H | $CH_3$ | 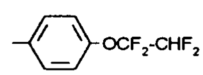 |
| 4-$OCHF_2CF_3$ | H | O | 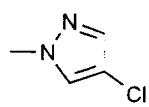 | H | H | H | $CH_3$ | 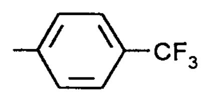 |
| 4-$OCHF_2CF_3$ | H | O | 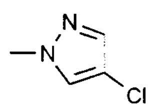 | H | H | H | $CH_3$ | 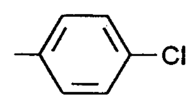 |
| 4-$CF_3$ | H | O | 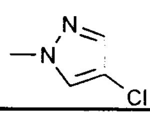 | H | H | H | $CH_3$ | 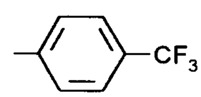 |
| 4-$CF_3$ | H | O | 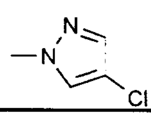 | H | H | H | $CH_3$ | 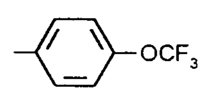 |
| H | H | O | 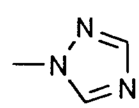 | H | H | H | $CH_3$ | 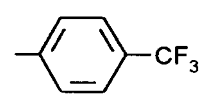 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-tert.-Butyl | H | O | 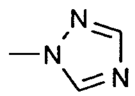 | H | H | H | $CH_3$ | 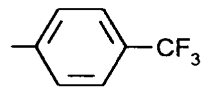 |
| 3-$CF_3$ | H | O | 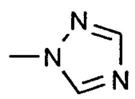 | H | H | H | $CH_3$ | 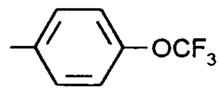 |
| 4-$CH_3$ | H | O | 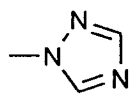 | H | H | H | $CH_3$ | 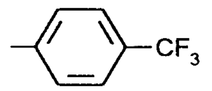 |
| 4-$CH_3$ | H | O | 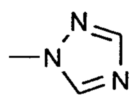 | H | H | H | $CH_3$ | 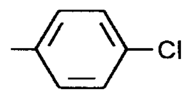 |
| 4-Cl | H | O | 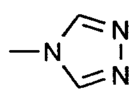 | H | H | H | $CH_3$ | 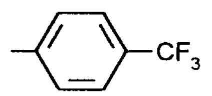 |
| 4-Cl | H | O | 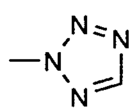 | H | H | H | $CH_3$ | 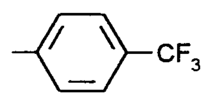 |
| 4-F | H | O | 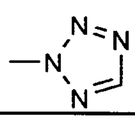 | H | H | H | $CH_3$ | 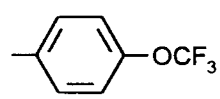 |
| 4-Cl | H | O | 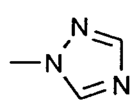 | H | $CH_3$ | H | $CH_3$ | 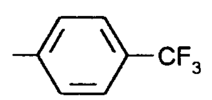 |
| 4-$OCHF_2$ | H | O | 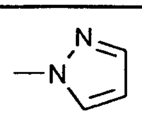 | H | $CH_3$ | H | $CH_3$ | 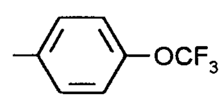 |
| 4-F | H | O | 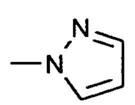 | H | $CH_3$ | $CH_3$ | $CH_3$ | 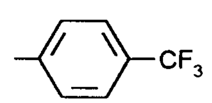 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 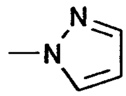 | H | H | H | $CH_3$ | 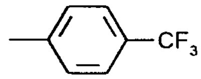 |
| 4-Cl | H | O | 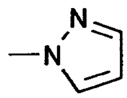 | H | H | H | $CH_3$ | 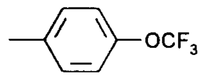 |
| 4-Cl | H | O | 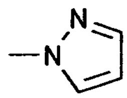 | H | H | H | $CH_3$ | 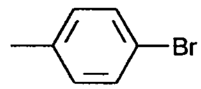 |
| 4-F | H | O | 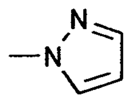 | H | H | H | $CH_3$ | 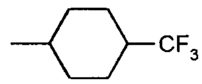 |
| 4-Br | H | O | 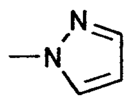 | H | H | H | $CH_3$ | 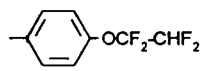 |
| 4-H | H | O | 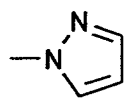 | H | H | H | $CH_3$ | 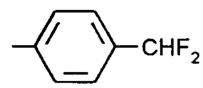 |
| 4-Cl | H | O | 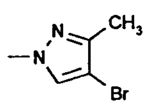 | H | H | H | $CH_3$ | 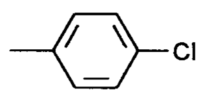 |
| 4-Cl | 3-Cl | O | 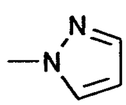 | $CH_3$ | H | H | $CH_3$ | 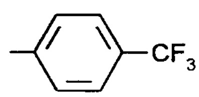 |
| 4-Cl | H | O | 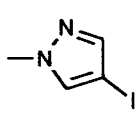 | H | H | H | $CH_3$ | 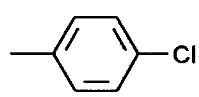 |

Tabelle 1 - Fortsetzung

| Y | Z | X | $R^1$ | $R^2$ | $R^3$ | $R^4$ | $R^5$ | $R^6$ |
|---|---|---|---|---|---|---|---|---|
| 4-Cl | H | O | 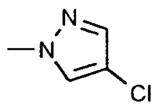 | H | H | H | $CH_3$ | 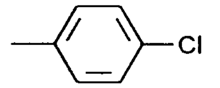 |
| 4-H | H | O | 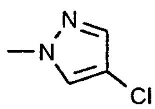 | H | H | H | $CH_3$ | 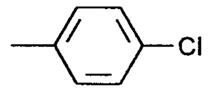 |
| 4-Cl | H | O | 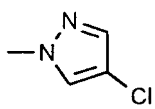 | H | H | H | $CH_3$ | 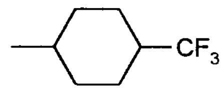 |
| 4-Cl | H | O | 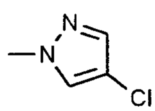 | H | H | H | $CH_3$ | 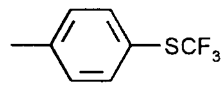 |
| 4-Cl | H | O | 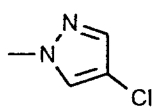 | H | H | H | $CH_3$ | 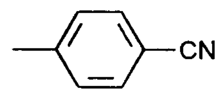 |
| 4-Cl | H | O | 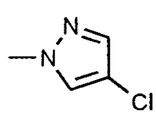 | H | H | H | $CH_3$ | 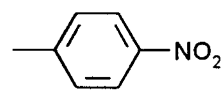 |
| 4-Cl | H | O | 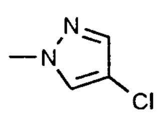 | H | H | H | $CH_3$ | 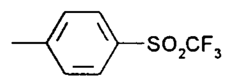 |
| 4-F | H | O | 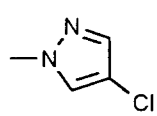 | H | H | H | $CH_3$ | 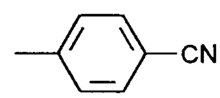 |
| 4-Cl | H | O | 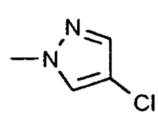 | H | H | H | $CH_3$ | 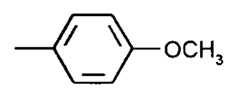 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 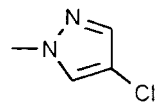 | H | H | H | $CH_3$ | 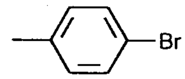 |
| 4-Cl | H | O | 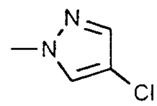 | H | H | H | $CH_3$ | 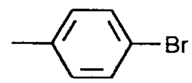 |
| 4-F | H | O | 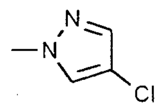 | H | H | H | $CH_3$ | 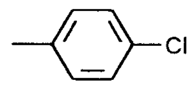 |
| 4-F | H | O | 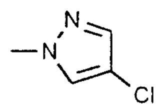 | H | H | H | $CH_3$ | 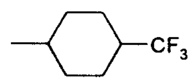 |
| 4-$OCH_3$ | H | O | 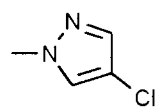 | H | H | H | $CH_3$ | 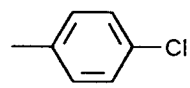 |
| 4-Br | H | O | 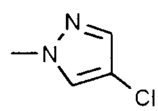 | H | H | H | $CH_3$ | 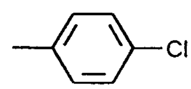 |
| 3,4- 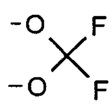 | | O | 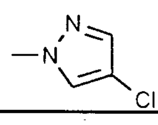 | H | H | H | $CH_3$ | 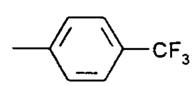 |
| 4-Cl | H | O | 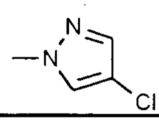 | H | H | $CH_3$ | $CH_3$ | 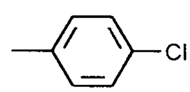 |
| 4-Cl | H | O | 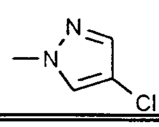 | iso-Prop | H | H | $CH_3$ | 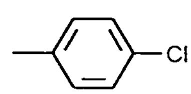 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-Cl | H | S | 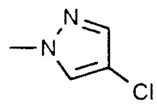 | H | H | H | $CH_3$ | 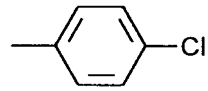 |
| 4-F | H | O | 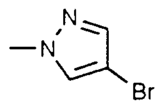 | H | H | H | $CH_3$ | 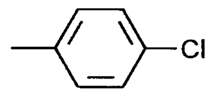 |
| 4-F | H | O | 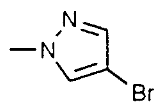 | H | H | H | $CH_3$ | 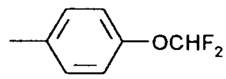 |
| 4-F | H | O | 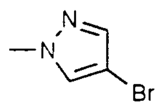 | H | H | H | $CH_3$ | 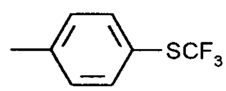 |
| 4-Cl | H | O | 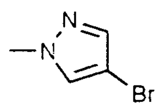 | H | H | H | $CH_3$ | 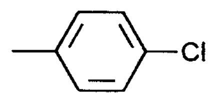 |
| 4-Cl | H | O | 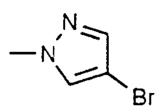 | H | H | H | $CH_3$ | 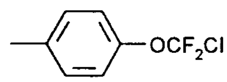 |
| 4-F | H | O | 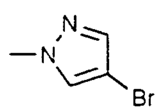 | H | H | H | $CH_3$ | 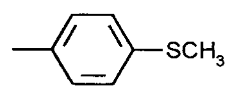 |
| 4-Cl | H | O | 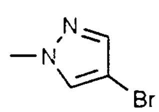 | H | H | H | $CH_3$ | 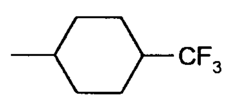 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 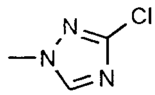 | H | H | H | $CH_3$ | 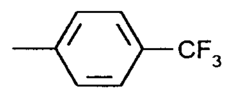 |
| 4-F | H | O | 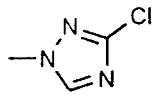 | H | H | H | $CH_3$ | 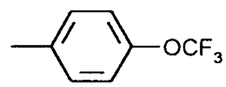 |
| 4-F | H | O | 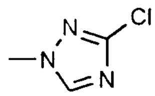 | H | H | H | $CH_3$ | 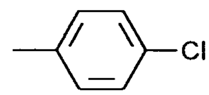 |
| 4-Cl | H | S | 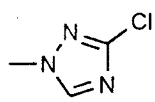 | H | H | H | $CH_3$ | 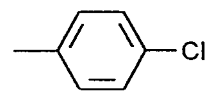 |
| 4-Cl | H | O | 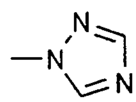 | H | H | H | $CH_3$ | 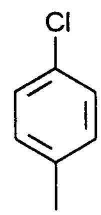 |
| 4-F | H | O | 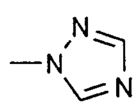 | H | H | H | $CH_3$ | 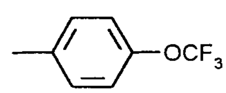 |
| 4-Br | H | S | 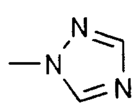 | H | H | H | $CH_3$ | 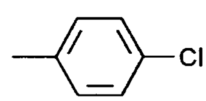 |
| 4-F | H | O | 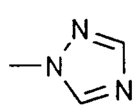 | H | H | H | $CH_3$ | 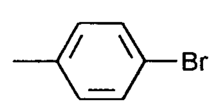 |
| 4-Cl | H | O | 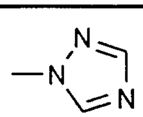 | H | H | H | $CH_3$ | 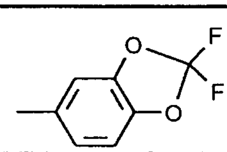 |

**Tabelle 1** - Fortsetzung

| Y | Z | X | $R^1$ | $R^2$ | $R^3$ | $R^4$ | $R^5$ | $R^6$ |
|---|---|---|---|---|---|---|---|---|
| 4-F | H | O | 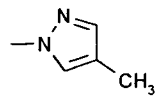 | H | H | H | $CH_3$ | 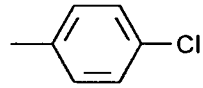 |
| 4-Cl | H | O | 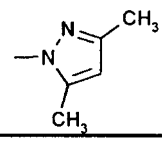 | H | H | H | $CH_3$ | 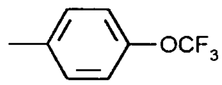 |
| 4-Cl | H | O | 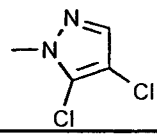 | H | H | H | $CH_3$ | 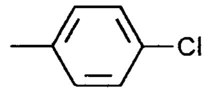 |
| 4-F | H | O | 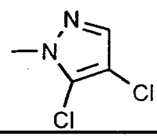 | H | H | H | $CH_3$ | 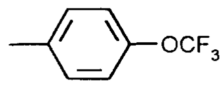 |
| 4-Cl | H | O | 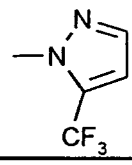 | H | H | H | $CH_3$ | 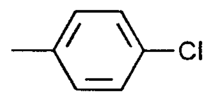 |
| 4-F | H | O | 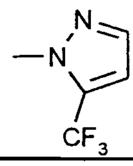 | H | H | H | $CH_3$ | 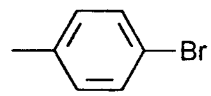 |
| 4-F | H | O | 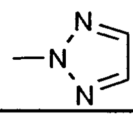 | H | H | H | $CH_3$ | 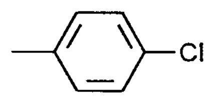 |
| 4-Cl | H | O | 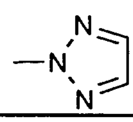 | H | H | H | $CH_3$ | 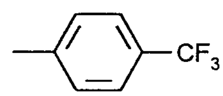 |
| 4-Cl | H | O | 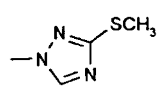 | H | H | H | $CH_3$ | 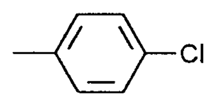 |

**Tabelle 2**

In Tabelle 2 sind die einzelnen Bedeutungen von Y, Z, X, $R^1$, $R^2$, $R^3$, $R^4$ und $R^6$ aufgelistet wie in Tabelle 1 und $R^5$ steht jeweils für $C_2H_5$.

**Tabelle 3**

In Tabelle 3 sind die einzelnen Bedeutungen von Y, Z, X, $R^1$, $R^2$, $R^3$, $R^4$ und $R^6$ aufgelistet wie in Tabelle 1 und $R^5$ steht jeweils für n-$C_3H_7$.

**Tabelle 4**

In Tabelle 4 sind die einzelnen Bedeutungen von Y, Z, X, $R^1$, $R^2$, $R^3$, $R^4$ und $R^6$ aufgelistet wie in Tabelle 1 und $R^5$ steht jeweils für i-$C_3H_7$.

**Tabelle 5**

In Tabelle 5 sind die einzelnen Bedeutungen von Y, Z, X, $R^1$, $R^2$, $R^3$, $R^4$ und $R^6$ aufgelistet wie in Tabelle 1 und $R^5$ steht jeweils für n-$C_4H_9$.

**Tabelle 6**

In Tabelle 6 sind die einzelnen Bedeutungen von Y, Z, X, $R^1$, $R^2$, $R^3$, $R^4$ und $R^6$ aufgelistet wie in Tabelle 1 und $R^5$ steht jeweils für $CO_2CH_3$.

**Tabelle 7**

In Tabelle 7 sind die einzelnen Bedeutungen von Y, Z, X, $R^1$, $R^2$, $R^3$, $R^4$ und $R^6$ aufgelistet wie in Tabelle 1 und $R^5$ steht jeweils für $CO_2C_2H_5$.

**Tabelle 8**

In Tabelle 8 sind die einzelnen Bedeutungen von Y, Z, X, $R^1$, $R^2$, $R^3$, $R^4$ und $R^6$ aufgelistet wie in Tabelle 1 und $R^5$ steht jeweils für $CH_2$-CH=$CH_2$.

## Tabelle 9

In Tabelle 9 sind die einzelnen Bedeutungen von Y, Z, X, $R^1$, $R^2$, $R^3$, $R^4$ und $R^6$ aufgelistet wie in Tabelle 1 und $R^5$ steht jeweils für $CH_2CO_2CH_3$.

## Tabelle 10

In Tabelle 10 sind die einzelnen Bedeutungen von Y, Z, X, $R^1$, $R^2$, $R^3$, $R^4$ und $R^6$ aufgelistet wie in Tabelle 1 und $R^5$ steht jeweils für $CH_2CH_2OCH_3$.

Verwendet man beispielsweise 3-(4'-Fluorphenyl)-4-(1"H-4"-chlorpyrazol-1"-yl)-4,5-dihydro-1-pyrazol-carbonsäure-(4-difluormethoxy)-anilid und 2-Iodpropan als Ausgangsstoffe und Natriumhydrid als Base, so läßt sich der Reaktionsablauf des erfindungsgemäßen Verfahrens a) durch das folgende Formelschema darstellen

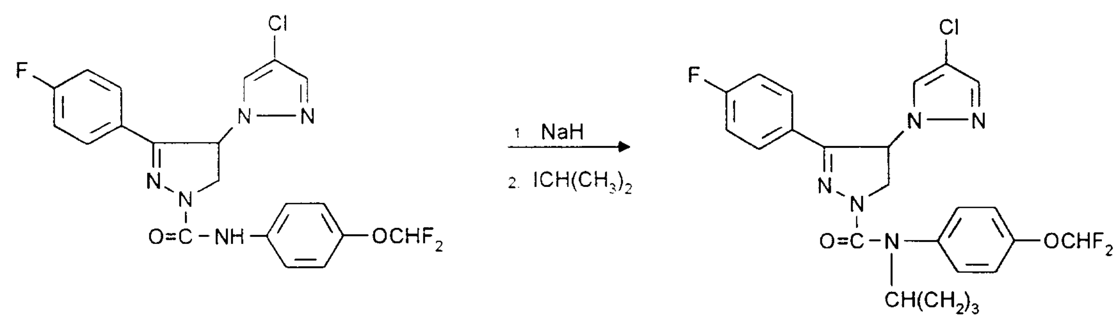


Verwendet man beispielsweise 3-(4'-Fluorphenyl)-4-(1"H-4"-chlorpyrazol-1"-yl)-4,5-dihydropyrazol und N-Methyl-4-chlorphenylcarbamidsäurechlorid als Ausgangsstoffe, so läßt sich der Reaktionsablauf des erfindungsgemäßen Verfahrens b) durch das folgende Formelschema darstellen

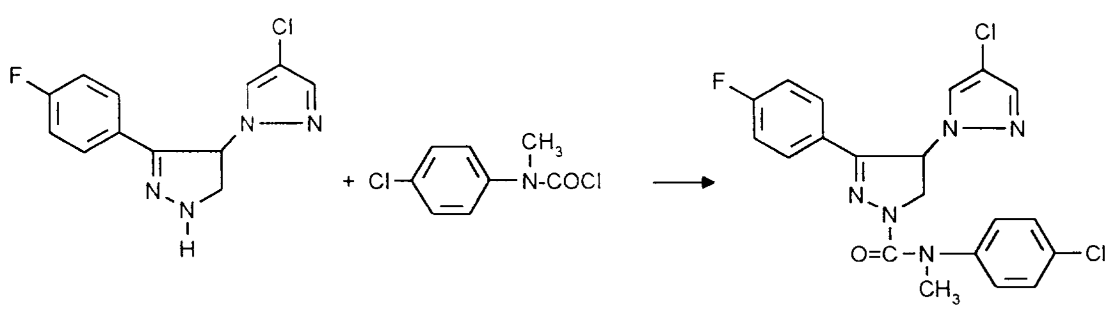

Die zur Durchführung des erfindungsgemäßen Verfahrens a) als Ausgangsstoffe benötigten substituierten Pyrazolinderivate sind durch die Formel (II) allgemein definiert. In dieser Formel (II) stehen $R^1$, $R^2$, $R^3$, $R^4$, $R^6$, Y und Z bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden. Die substituierten Pyrazolinderivate der Formel (II) sind bekannt und/oder erhältlich nach bekannten Verfahren (vgl. EP-A-0 438 690).

Die ebenfalls zur Durchführung des erfindungsgemäßen Verfahrens a) als Ausgangsstoffe benötigten Alkylierungs- bzw. Acylierungsmittel sind durch die Formel (III) allgemein definiert. In dieser Formel (III) steht $R^5$ bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für

diesen Substituenten genannt wurden.

A steht für eine Abgangsgruppe, vorzugsweise für Halogen, insbesondere Chlor, Brom oder Iod, oder für jeweils gegebenenfalls substituiertes Alkylsulfonyloxy, Alkoxysulfonyloxy, oder Arylsulfonyloxy wie insbesondere Methansulfonyloxy, Trifluormethansulfonyloxy, Methoxysulfonyloxy, Ethoxysulfonyloxy oder p-Toluolsulfonyloxy.

Die Verbindungen der Formel (III) sind allgemein bekannte Synthesechemikalien der organischen Chemie.

Die zur Durchführung des erfindungsgemäßen Verfahrens b) als Ausgangsstoffe benötigten substituierten Pyrazoline sind durch die Formel (IV) allgemein definiert. In dieser Formel (IV) stehen $R^1$, $R^2$, $R^3$, $R^4$, Y und Z bevorzugt bzw. besonders bevorzugt für diejenigen Reste, die bereits im Zusammenhang mit der Beschreibung der erfindungsgemäßen Stofe der Formel (I) als bevorzugt bzw. als besonders bevorzugt für diese Substituenten genannt wurden. Die substituierten Pyrazoline der Formel (IV) sind bekannt und/oder erhältlich nach bekannten Verfahren (vgl. EP-A-0 438 690 und EP-A-0 546 420).

Die ebenfalls zur Durchführung des erfindungsgemäßen Verfahrens b) als Ausgangsstoffe benötigten Carbamidsäurechloride sind durch die Formel (V) allgemein definiert.

In dieser Formel (V) stehen $R^5$ und $R^6$ bevorzugt bzw. besonders bevorzugt für diejenigen Reste, die bereits im Zusammehang mit der Beschreibung der erfindungsgemäßen Stoffe der Formel (I) als bevorzugt bzw. besonders bevorzugt für diese Substituenten genannt wurden. Die Carbamidsäurechloride der Formel (V) sind allgemein bekannte Verbindungen der organischen Chemie. Man erhält sie beispielsweise durch Umsetzung der entsprechend substituierten Amine mit Phosgen.

Das Verfahren a) ist dadurch gekennzeichnet, daß man Verbindungen der Formel (II), in welcher $R^1$, $R^2$, $R^3$, $R^4$, $R^6$, X, Y und Z die oben angegebene Bedeutung haben, in Gegenwart einer Base und gegebenenfalls in Gegenwart eines Verdünnungsmittels mit Verbindungen der Formel (III), in welcher $R^5$ und A die oben angegebene Bedeutung haben, umsetzt.

Als Basen können bei der Durchführung des erfindungsgemäßen Verfahrens a) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid. Adogen 464 (= Methyltrialkyl($C_8$-$C_{10}$)-ammoniumchlorid) oder TDA 1 (= Tris-(methoxyethoxyethyl)-amin) eingesetzt werden können.

Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Als Verdünnungsmittel können bei dem erfindungsgemäßen Verfahren a) alle inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei dem erfindungsgemäßen Verfahren a) innerhalb eines größeren Bereichs variiert werden. Im allgemeinen arbeitet man bei Temperaturen zwischen -20 und 150 °C, vorzugsweise zwischen 0 und 100 °C.

Die Ausgangsverbindungen der Formel (II) und der Formel (III) werden im allgemeinen in äquimolaren Mengen eingesetzt. Es ist jedoch auch möglich, einen Überschuß an der Verbindung der Formel (III) einzusetzen.

Die Base wird beim erfindungsgemäßen Verfahren a) im allgemeinen in mindestens äquimolaren Mengen zugesetzt.

Es wird vorzugsweise bei Normaldruck gearbeitet.

Das Verfahren b) ist dadurch gekennzeichnet, daß man Verbindungen der Formel (IV), in welcher $R^1$, $R^2$, $R^3$, $R^4$, Y und Z die oben angegebene Bedeutung haben, mit Carbamidsäurechloriden der Formel (V), in welcher $R^5$ und $R^6$ die oben angegebene Bedeutung haben, gegebenenfalls in Gegenwart einer Base und gegebenenfalls in Gegenwart eines Verdünnungsmittels umsetzt.

Als Basen kommen beim erfindungsgemäßen Verfahren b) alle üblichen anorganischen oder organischen Basen in Frage, beispielhaft seien Natriumhydroxid, Natriumcarbonat, Kaliumcarbonat, Triethylamin oder Pyridin genannt.

Als Verdünnungsmittel kommen alle inerten polaren organischen Lösungsmittel in Frage wie Kohlenwasserstoffe, Ether, Amide, Sulfone oder Sulfoxide, vorzugsweise werden Toluol, Dimethylsulfoxid, Tetrahydrofuran, Dimethylformamid oder Methylenchlorid eingesetzt.

Man setzt pro Mol Ausgangsverbindung der Formel (IV) ca. 1 Mol Carbamidsäurechlorid der Formel (V) bei 0 bis 150 °C, vorzugsweise bei 20 bis 70 °C um.

Es wird vorzugsweise bei Normaldruck gearbeitet.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci. Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Phylloxera vastatrix, Pemphigus spp., Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Orvzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp.,

Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) einsetzen.

Weiterhin zeigen die erfindungsgemäßen Verbindungen eine Wirksamkeit gegenüber parasitischen Protozoen und zwar insbesondere gegen Coccidien und/oder Plasmodium.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith. Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Feilsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfärbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:

Acrinathrin, Alphamethrin, Betacyfluthrin, Bifenthrin, Brofenprox, Cis-Resmethrin, Clocythrin, Cycloprothrin,

Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zeta-methrin, Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb, Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliplios, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, Buprofezin, Chlorfluazuron, Diflubenzuron, Flucycloxuron, Flufenoxuron, Hexaflumuron, Pyriproxifen, Tebufenozide, Teflubenzuron, Triflumuron, Imidacloprid, Nitenpyram, N-[(6-Chloro-3-pyridinyl)methyl]-N'-cyano-N-methyl-ethanimidamid (NI-25), Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron, Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox, Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad. Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die Herstellung und die biologische Wirksamkeit der erfindungsgemäßen Verbindungen soll anhand der folgenden Beispiele erläutert werden.

## Herstellungsbeispiele

### Beispiel 1

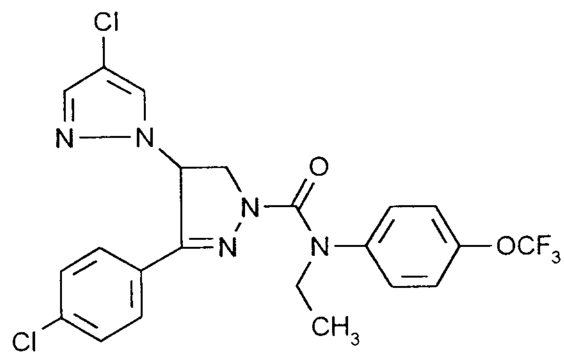

5 g (3-(4'-Chlorphenyl)-4-(1"H-4"-chlorpyrazol-1"-yl)-4,5-dihydro-1-pyrazol-carbonsäure-(4-trifluormethoxy)-anilid werden in 50 ml Dimethylformamid gelöst und bei 20 bis 40 ° C unter Rühren mit 0,5 g Natriumhydrid versetzt. Anschließend fügt man 2,8 g Ethylbromid hinzu und erwärmt 45 Minuten auf 80 ° C. Nach dem Abkühlen wird die Reaktionsmischung in 150 ml Wasser gegossen und dann mit je 2 x 100 ml Chloroform extrahiert. Die vereinigte organische Phase wird über Magnesiumsulfat getrocknet und anschließend im Vakuum eingeengt. Der verbleibende kristalline Rückstand wird aus Ethanol umkristallisiert. Man erhält 0,9 g 3-(4'-Chlorphenyl)-4-(1"H-4"-chlorpyrazol-1"-yl)-4,5-dihydro-1-pyrazolcarbonsäure-(4-trifluormethoxy)-N-ethyl-anilid als farblose Kristalle mit dem Schmp.: 153 ° C. Alternativ zur Umkristallisation kann das Rohprodukt auch über Kieselgel chromatographiert werden mit dem Laufmittel n-Hexan/Aceton 7:3.

### Beispiel 2

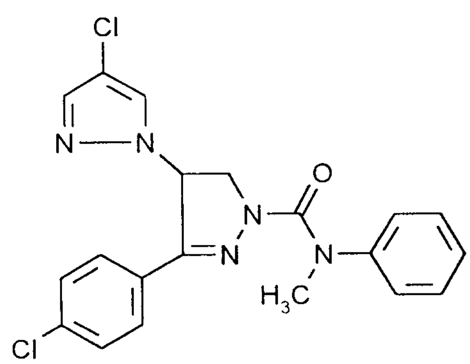

2,8 g 3-(4'-Chlorphenyl)-4-(1"H-4"-chlorpyrazol-1"-yl)-4,5-dihydro-1-H-pyrazolin werden in 50 ml wasserfreiem Toluol gelöst und mit 1 g Pyridin versetzt. Bei 50 ° C werden 1,7 g N-Methyl-N-phenyl-carbamidsäurechlorid hinzugegeben. Man rührt anschließend 30 Minuten bei 40 ° C und 10 Stunden bei 20 ° C. Das Reaktionsgemisch wird dann in 100 ml Wasser gegossen, die organische Phase abgetrennt, über Magnesiumsulfat getrocknet und im Vakuum eingeent. Man erhält 2 g 3-(4'-Chlorphenyl)-4-(1"H-4"-chlorpyrazol-1"-yl)-4,5-dihydro-1-pyrazolcarbonsäure-N-methyl-anilid als zähes Öl.

Analog zu den Beispielen 1 und 2 und gemäß den allgemeinen Angaben zur Herstellung werden die in der Tabelle 2 genannten Verbindungen der Formel (I) hergestellt, wobei die Reste Z, $R^2$, $R^3$ und $R^4$ jeweils für Wasserstoff stehen und X für Sauerstoff steht:

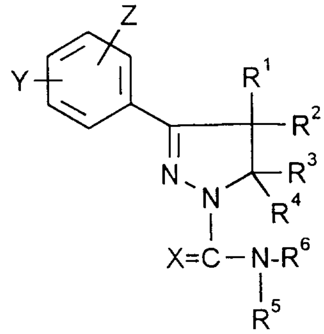

(I)

Tabelle 2

| Bsp. Nr. | Y | $R^1$ | $R^5$ | $R^6$ | physik. Eigenschaften |
|---|---|---|---|---|---|
| 3 | 4-Cl | 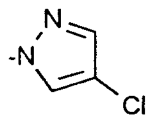 | $C_2H_5$ | 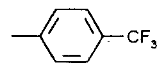 | Fp. 170°C |
| 4 | 4-Cl | 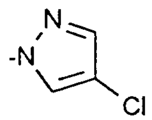 | $C_2H_5$ | 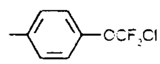 | Fp. 135°C |
| 5 | 4-Cl | 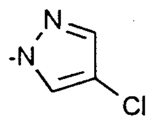 | $CH_2CO_2C_2H_5$ | 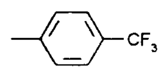 | Fp. 78°C |
| 6 | 4-Cl | 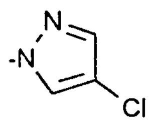 | n-$C_4H_9$ | 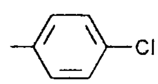 | Fp. 158°C |
| 7 | 4-Cl | 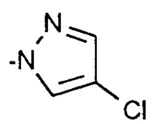 | n-$C_3H_7$ | 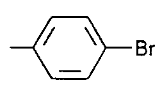 | Fp. 150°C |
| 8 | 4-Cl | 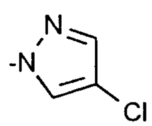 | n-$C_4H_9$ | 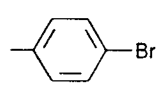 | Fp. 158°C |
| 9 | 4-Cl | 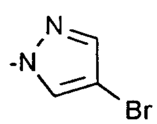 | $C_2H_5$ | 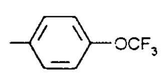 | Fp. 171°C |

**Tabelle 2** - Fortsetzung

| Bsp. Nr. | Y | $R^1$ | $R^5$ | $R^6$ | physik. Eigenschaften |
|---|---|---|---|---|---|
| 10 | 4-Cl | 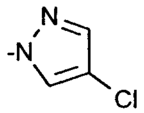 | $CH_3$ | 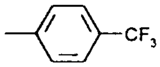 | Fp. 191°C |
| 11 | 4-Cl | 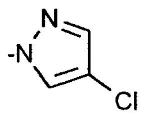 | $CH_2$-CH=$CH_2$ | 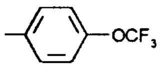 | Fp. 135°C |
| 12 | 4-F | 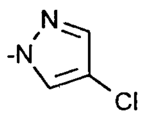 | $C_2H_5$ | 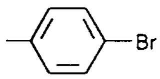 | Fp. 127°C |
| 13 | 4-Cl | 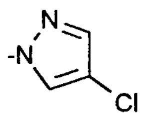 | $C_2H_5$ | 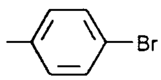 | Fp. 176°C |
| 14 | 4-F | 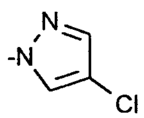 | n-$C_3H_7$ | 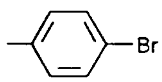 | Fp. 114°C |
| 15 | H | 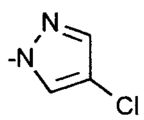 | $CH_3$ | 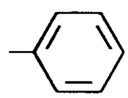 | Öl |
| 16 | 4-F | 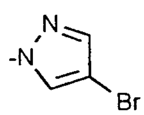 | $CH_3$ | 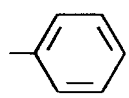 | Öl |
| 17 | 4-$OCHF_2$ | 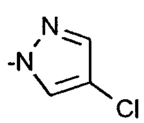 | $CH_3$ | 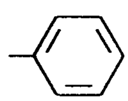 | Öl |

Tabelle 2 - Fortsetzung

| Bsp. Nr. | Y | $R^1$ | $R^5$ | $R^6$ | physik. Eigenschaften |
|---|---|---|---|---|---|
| 18 | 4-Br | 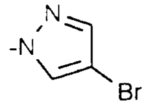 | $CH_3$ | 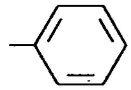 | Öl |
| 19 | 4-F | 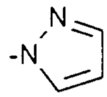 | $CH_3$ | 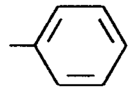 | Öl |
| 20 | H | 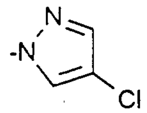 | CN | 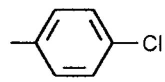 | Fp. 153°C |
| 21 | 4-F | 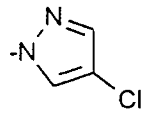 | CN | 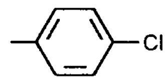 | Fp. 196°C |
| 22 | 4-Cl | 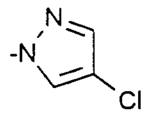 | CN | 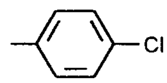 | Fp. 178°C |
| 23 | $4\text{-}OCHF_2$ | 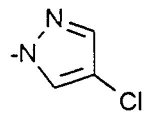 | CN | 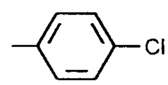 | Fp. 184°C |
| 24 | 4-F | 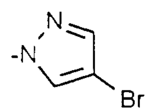 | CN | 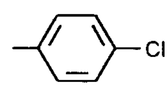 | Fp. 198°C |
| 25 | 4-Br | 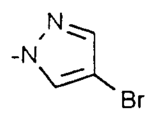 | CN | 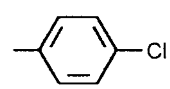 | Fp. 196°C |

**Tabelle 2** - Fortsetzung

| Bsp. Nr. | Y | $R^1$ | $R^5$ | $R^6$ | physik. Eigenschaften |
|---|---|---|---|---|---|
| 26 | 4-F | 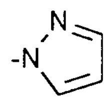 | CN | 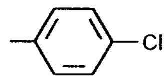 | Fp. 172°C |
| 27 | 4-Cl | 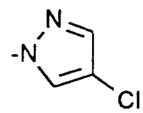 | $CH_3$ | 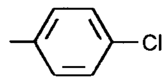 | Fp. 169°C |
| 28 | 4-Cl | 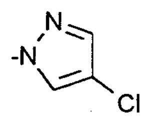 | $C_2H_5$ | 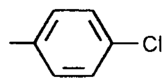 | Fp. 154°C |
| 29 | 4-Cl | 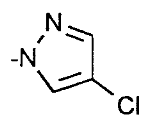 | n-$C_3H_7$ | 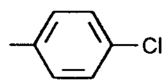 | |
| 30 | 4-Cl | 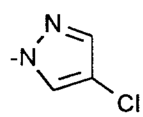 | n-$C_4H_9$ | 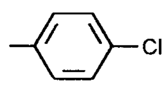 | |
| 31 | 4-Cl | 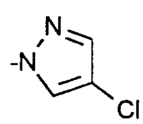 | i-$C_3H_7$ | 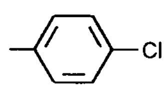 | Fp. 182°C |
| 32 | 4-Cl | 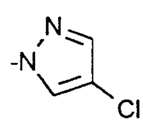 | $CH_2CO_2C_2H_5$ | 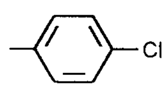 | |
| 33 | 4-Cl | 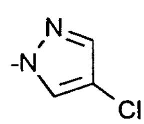 | $CH_2$-CH=$CH_2$ | 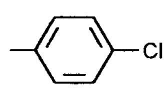 | |

**Tabelle 2** - Fortsetzung

| Bsp. Nr. | Y | $R^1$ | $R^5$ | $R^6$ | physik. Ei- genschaften |
|---|---|---|---|---|---|
| 34 | 4-Cl | 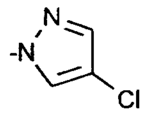 | 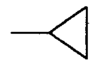 | 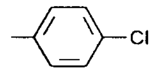 | |
| 35 | 4-Cl | 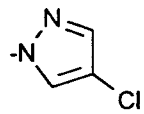 | $CHF_2$ | 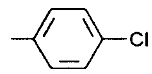 | |
| 36 | 4-Cl | 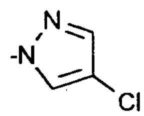 | $CH_2CH_2OCH_3$ | 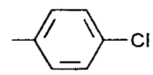 | |
| 37 | 4-Cl | 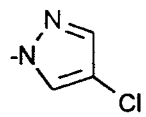 | $CH_2$-C≡CH | 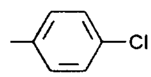 | |
| 38 | 4-Cl | 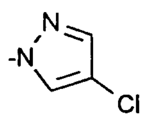 | $CH_2CH_2N(CH_3)_2$ | 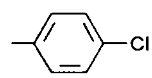 | |
| 39 | 4-Cl | 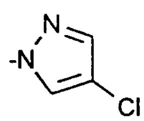 | $CH_2COCH_3$ | 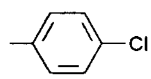 | |
| 40 | 4-Cl | 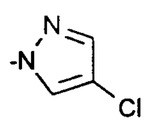 | $CO_2CH_3$ | 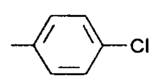 | |
| 41 | 4-Cl | 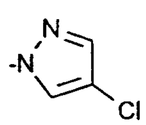 | 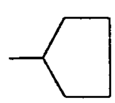 | 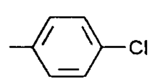 | |

**Tabelle 2** - Fortsetzung

| Bsp. Nr. | Y | $R^1$ | $R^5$ | $R^6$ | physik. Eigenschaften |
|---|---|---|---|---|---|
| 42 | 4-Cl | 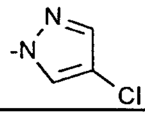 | s-$C_4H_9$ | 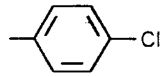 | |
| 43 | 4-Cl | 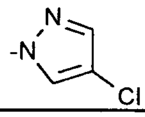 | n-$C_3H_7$ | 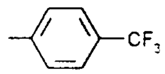 | |
| 44 | 4-Cl | 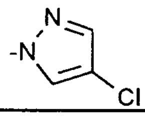 | $CH_2CH{=}CH_2$ | 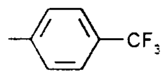 | |
| 45 | 4-Cl | 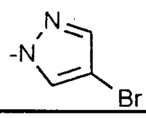 | $C_2H_5$ | 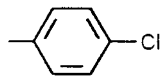 | |
| 46 | 4-Cl | 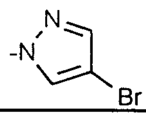 | n-$C_3H_7$ | 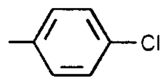 | |
| 47 | 4-Cl | 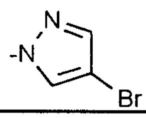 | $CH_3$ | 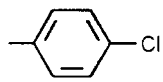 | Fp. 164°C |
| 48 | 4-Cl | 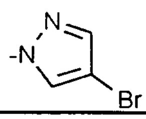 | i-$C_3H_7$ | 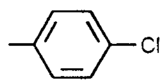 | Fp. 198°C |
| 49 | 4-Cl | 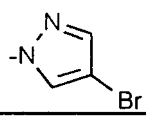 | $CH_2CO_2C_2H_5$ | 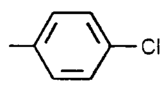 | |
| 50 | 4-Cl | 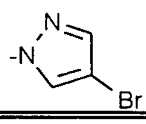 | $CH_2$-$CH{=}CH_2$ | 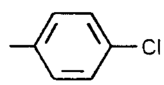 | |

**Tabelle 2** - Fortsetzung

| Bsp. Nr. | Y | $R^1$ | $R^5$ | $R^6$ | physik. Eigenschaften |
|---|---|---|---|---|---|
| 51 | 4-F | 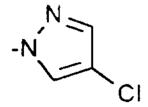 | $CH_3$ | 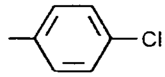 | |
| 52 | 4-F | 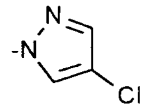 | $C_2H_5$ | 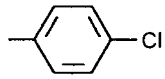 | |
| 53 | 4-F | 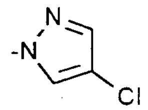 | $n-C_3H_7$ | 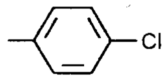 | |
| 54 | 4-F | 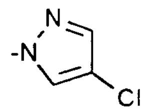 | $i-C_3H_7$ | 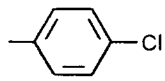 | |
| 55 | 4-F | 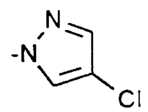 | $n-C_4H_9$ | 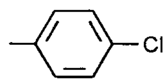 | |
| 56 | 4-F | 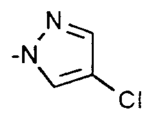 | $CH_2C{\equiv}CH$ | 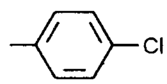 | |
| 57 | 4-F | 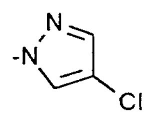 | $CH_2CO_2C_2H_5$ | 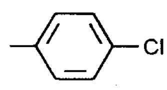 | |
| 58 | 4-F | 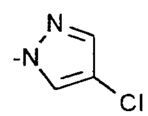 | $CH_2-CH=CH_2$ | 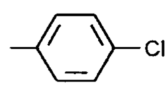 | |

**Tabelle 2**-Forsetzung

| Bsp. Nr. | Y | $R^1$ | $R^5$ | $R^6$ | physik. Eigenschaften |
|---|---|---|---|---|---|
| 59 | 4-Cl | 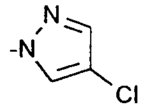 | $CH_3$ | 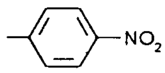 | Fp. 172°C |
| 60 | 4-Cl | 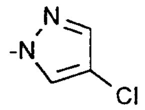 | $C_2H_5$ | 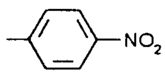 | |
| 61 | 4-F | 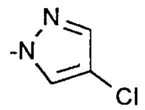 | $CH_3$ | 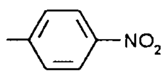 | |
| 62 | 4-Cl | 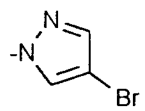 | $CH_3$ | 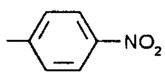 | Fp.172°C |
| 63 | 4-Cl | 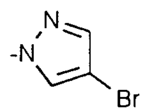 | n-$C_4H_9$ | 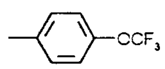 | Fp. 101°C |
| 64 | 4-Br | 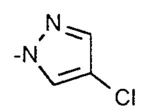 | $CH_3$ | 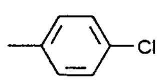 | Fp. 184°C |
| 65 | 4-Br | 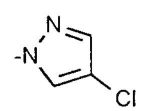 | $CH_3$ | 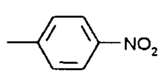 | Fp. 165°C |
| 66 | 4-$OCHF_2$ | 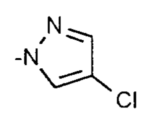 | $CH_3$ | 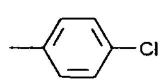 | Fp. 176°C |

**Tabelle 2**-Forsetzung

| Bsp. Nr. | Y | $R^1$ | $R^5$ | $R^6$ | physik. Eigenschaften |
|---|---|---|---|---|---|
| 67 | 4-$OCHF_2$ | 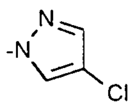 | $CH_3$ | 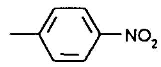 | Fp. 192°C |
| 68 | 4-$OCHF_2$ | 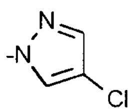 | $CH_3$ | 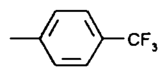 | Fp. 172°C |
| 69 | 4-Br | 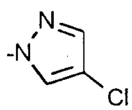 | $CH_3$ | 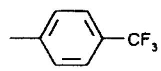 | Fp. 174°C |
| 70 | 4-Cl | 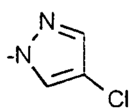 | $CH_3$ | 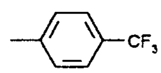 | Fp. 188°C |
| 71 | 4-Br | 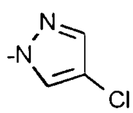 | $C_2H_5$ | 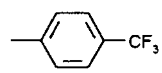 | Fp. 177°C |
| 72 | 4-Br | 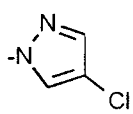 | $C_2H_5$ | 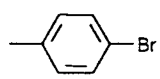 | Fp. 172°C |
| 73 | 4-Cl | 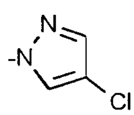 | n-$C_6H_{13}$ | 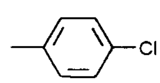 | Öl |
| 74 | 4-Cl | 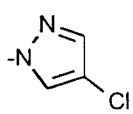 | $-CH_2-C\equiv CH$ | 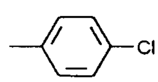 | Fp. 190°C |

In den folgenden Anwendungsbeispielen wurden die aus der EP-A 0 438 690 bekannten Verbindungen (A) und (B) als Vergleichssubstanz eingesetzt.

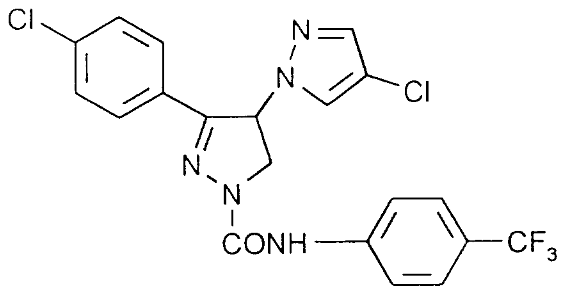

(A)

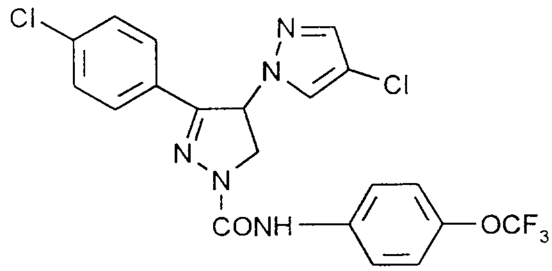

(B)

**Anwendungsbeispiele**

**Beispiel A**

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochhleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen 10, 20 und 24 bei einer beispielhaften Wirkstoffkonzentration von 0,01 % eine Abtötung von 100 % nach 3 Tagen.

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besitzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen 10, 3 und 4 bei einer beispielhaften Wirkstoffkonzentration von 0,001 % eine Abtötung von 100 % nach 7 Tagen.

**Beispiel C**

Heliothis virescens-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis cirescens) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel 10 bei einer beispielhaften Wirkstoffkonzentration von 0,0001 % eine Abtötung von 90 % nach 7 Tagen.

**Beispiel D**

Nephotettix-Test

Lösungsmittel: 7 Gew.-Teile Dimethylformamid
Emulgator: 1 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen 1 und 4 bei einer beispielhaften Wirkstoffkonzentration von 0,01 % eine Abtötung von mindestens 80 % nach 6 Tagen.

## Patentansprüche

**1.** Substituierte Pyrazolinderivate der Formel (I)

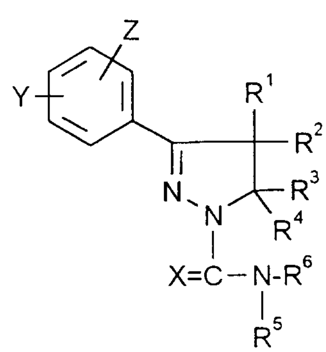

(I)

in welcher

$R^1$ für einen ungesättigten fünfgliedrigen, 1 bis 4 Stickstoffatome enthaltenden, gegebenenfalls substituierten und gegebenenfalls benzokondensierten Heterocyclus steht,

$R^2$ für Wasserstoff, Alkyl, gegebenenfalls substituiertes Cycloalkyl, Halogenalkyl, Halogenalkylthio, Alkoxycarbonyl oder Trialkylsilyl steht,

$R^3$ für Wasserstoff oder Alkyl steht,

$R^4$ für Wasserstoff oder Alkyl steht,

$R^5$ für Cyano, für jeweils gegebenenfalls substituiertes Alkyl, Cycloalkyl, Alkenyl oder Alkinyl, für Formyl, für jeweils gegebenenfalls substituiertes Alkylcarbonyl oder Alkoxycarbonyl steht,

$R^6$ für gegebenenfalls substituiertes Alkyl, gegebenenfalls substituiertes Cycloalkyl oder für den Rest

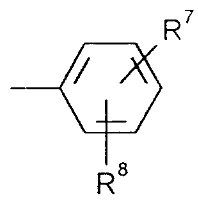

steht,

wobei $R^7$ und $R^8$ gleich oder verschieden sein können und für Wasserstoff, Halogen, Alkyl, Nitro, Cyano, Halogenalkyl, Alkoxy, Halogenalkoxy, Alkylthio, Halogenalkylthio, jeweils gegebenenfalls substituiertes Phenoxy oder Phenylthio, gegebenenfalls substituiertes Mono- oder Dialkylamino, gegebenenfalls substituiertes Cycloalkyl, Alkoxycarbonyl, Alkenyl, Alkinyl, Alkylthionyl, Alkylsulfonyl, Halogenalkylthionyl, Halogenalkylsulfonyl, Halogenalkoxycarbonyl oder wobei $R^7$ und $R^8$ zusammen für einen bivalenten gegebenenfalls ein oder zwei Sauerstoffatome enthaltenden und gegebenenfalls substituierten Rest stehen,

X für Sauerstoff oder Schwefel steht und

Y und Z unabhängig voneinander für Wasserstoff, Alkyl, Halogen, Halogenalkyl, Alkoxy, Alkylthio, Halogenalkoxy, Halogenalkylthio, Alkoxycarbonyl, Halogenalkoxycarbonyl, jeweils gegebenenfalls substituiertes Aryloxy oder Arylthio, Alkenyl, Alkinyl, Alkylthionyl, Alkylsulfonyl, Halogenalkylthionyl, Halogenalkylsulfonyl, Amino, gegebenenfalls substituiertes Mono- oder Dialkylamino, Nitro, Cyano stehen oder Y und Z zusammen für gegebenenfalls durch Halogen substituiertes 3,4-Methylendioxy oder 3,4-Ethylendioxy stehen.

**2.** Substituierte Pyrazolinderivate der Formel (I) gemäß Anspruch 1, in welcher

$R^1$ für einen gegebenenfalls einfach oder mehrfach gleich oder verschieden durch Halogen, Alkyl($C_1$-$C_6$), CN, $NO_2$, Alkoxy($C_1$-$C_6$)carbonyl, Alkyl($C_1$-$C_4$)thio, Alkoxy($C_1$-$C_6$), Halogenalkyl($C_1$-$C_4$), Halogenalkyl($C_1$-$C_4$)thio, Halogenalkoxy($C_1$-$C_4$), Amino, Mono- oder Dialkyl($C_1$-$C_4$)amino oder Halogenalkoxy($C_1$-$C_6$)carbonyl substituierten 1H-Pyrrolyl-(1)-, 1H-Pyrazolyl-(1)-, 1H-Imidazolyl-(1)-, 2H-1,2,3,-Triazolyl-(2)-, 1H-1,2,3-Triazolyl-(1)-, 1H-1,2,4,-Triazolyl-(1)-, 4H-1,2,4-Triazolyl-(4)-, 2H-Tetrazolyl-(2)- oder 1H-Tetrazolyl-(1)-Rest steht,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$): Halogenalkyl($C_1$-$C_4$), Halogenalkyl($C_1$-$C_4$)thio, Alkoxy($C_1$-$C_6$)carbonyl oder Trialkyl($C_1$-$C_6$)silyl steht,

$R^3$ für Wasserstoff oder Alkyl($C_1$-$C_6$) steht,

$R^4$ für Wasserstoff oder Alkyl($C_1$-$C_6$) steht,

$R^5$ für Cyano, für gegebenenfalls durch Halogen, Alkoxy($C_1$-$C_6$) oder einen der Reste

$CO_2R^7$ oder $NR^8R^9$

substituiertes Alkyl($C_1$-$C_{10}$) steht,

wobei

$R^7$ für gegebenenfalls durch Halogen substituiertes Alkyl($C_1$-$C_{10}$) steht, $R^8$ und $R^9$ gleich oder verschieden sind und für Alkyl($C_1$-$C_{10}$) stehen oder

$R^8$ und $R^9$ gemeinsam mit dem Stickstoffatom, an das sie gebunden sind einen 3- bis 8-gliedrigen Ring bilden, der gegebenenfalls ein weiteres Heteroatom enthält, für gegebenenfalls durch Halogen oder Halogenalkyl($C_1$-$C_6$) substituiertes Cycloalkyl($C_3$-$C_8$), für jeweils gegebenenfalls durch Halogen oder Alkoxy($C_1$-$C_6$) substituiertes Alkenyl($C_2$-$C_8$) oder Alkinyl($C_2$-$C_8$), für Formyl, für gegebenenfalls durch Halogen substituiertes Alkyl($C_1$-$C_{10}$)carbonyl oder für gegebenenfalls durch Halogen substituiertes Alkoxy($C_1$-$C_{10}$)carbonyl steht,

$R^6$ für gegebenenfalls durch Halogen oder Halogenalkoxy($C_1$-$C_4$) substituiertes Alkyl($C_1$-$C_6$),

für gegebenenfalls durch Halogen, Halogenalkyl($C_1$-$C_4$), Halogenalkoxy($C_1$-$C_4$) substi-

tuiertes Cycloalkyl($C_3$-$C_7$) oder für den Rest

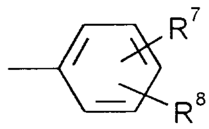

steht,
wobei $R^7$ und $R^8$ gleich oder verschieden sein können und für Wasserstoff, Halogen, Alkyl($C_1$-$C_6$), Nitro, Cyano, Halogenalkyl($C_1$-$C_6$), Alkoxy($C_1$-$C_6$), Halogenalkoxy($C_1$-$C_6$), Alkyl($C_1$-$C_6$)thio, Halogenalkyl($C_1$-$C_6$)thio, jeweils gegebenenfalls durch Halogen, Halogenalkyl($C_1$-$C_6$), Alkoxy($C_1$-$C_6$), Alkyl($C_1$-$C_6$) substituiertes Phenoxy oder Phenylthio, gegebenenfalls durch Halogen, Alkoxy($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$), Alkinyl($C_2$-$C_6$), Alkyl($C_1$-$C_4$)thionyl, Alkyl-($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei
$R^7$ und $R^8$ zusammen für einen der folgenden bivalenten Reste stehen:

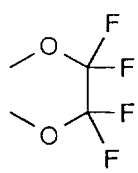 , 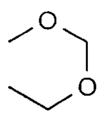 , 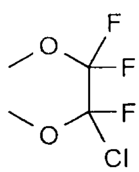 , 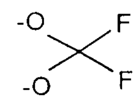 .

X — für Sauerstoff oder Schwefel steht,
Y und Z — unabhängig voneinander für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_6$), Halogenalkyl($C_1$-$C_6$)thio, Alkoxy($C_1$-$C_4$)-carbonyl, Halogenalkoxy($C_1$-$C_4$)carbonyl, jeweils gegebenenfalls durch Halogen, Alkyl($C_1$-$C_4$), Alkoxy($C_1$-$C_4$), Halogenalkyl($C_1$-$C_4$) substituiertes Phenoxy oder Phenylthio, Alkenyl($C_2$-$C_6$), Alkinyl($C_2$-$C_6$), Alkyl($C_1$-$C_4$)-thionyl, Alkyl($C_1$-$C_4$)-sulfonyl, Halogenalkyl($C_1$-$C_4$)thionyl, Halogenalkyl($C_1$-$C_4$)sulfonyl, gegebenenfalls durch Halogen, Alkoxy($C_1$-$C_4$) substituiertes Mono- oder Dialkyl($C_1$-$C_6$)amino, Nitro oder Cyano stehen oder
Y und Z — gemeinsam für gegebenenfalls durch Fluor und/oder Chlor substituiertes 3,4-Methylendioxy oder 3,4-Ethylendioxy stehen.

**3.** Substituierte Pyrazolenderivate der Formel (I) gemäß Anspruch 1, in welcher
$R^1$ — für einen gegebenenfalls ein- oder zweifach, gleich oder verschieden durch Fluor, Chlor, Brom, Jod, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl($C_1$-$C_3$), Halogenalkyl($C_1$-$C_3$)thio, Amino oder Dialkyl($C_1$-$C_3$)aminooder Halogenalkoxy($C_1$-$C_4$)carbonyl substituierten 1-H-Pyrrolyl-(1)-, 1H-Pyrazolyl-(1)-, 1H-Imidazolyl-(1)-, 2H-1,2,3-Triazolyl-(2)-, 1H-1,2,3-Triazolyl-(1)-, 1H,1,2,4-Triazolyl-(1)-, 4H-1,2,4-Triazolyl-(4)-, 2H-Tetrazolyl-(2) oder 1H-Tetrazolyl-(1)-Rest steht,
$R^2$ — für Wasserstoff, Alkyl($C_1$-$C_4$), gegebenenfalls durch Fluor, Chlor, Brom oder Halogenalkyl($C_1$-$C_3$) substituiertes Cycloalkyl ($C_3$-$C_6$), Halogenalkyl($C_1$-$C_3$), Halogenalkyl($C_1$-$C_3$)thio, Alkoxy($C_1$-$C_4$)carbonyl oder Trialkyl($C_1$-$C_4$)silyl steht,
$R^3$ — für Wasserstoff oder Alkyl($C_1$-$C_4$) steht,
$R^4$ — für Wasserstoff oder Alkyl($C_1$-$C_4$)steht,
$R^5$ — für Cyano, für gegebenenfalls durch Fluor, Chlor, Brom, Alkoxy($C_1$-$C_4$) oder einen der Reste
$CO_2R^7$ oder $NR^8R^9$
substituiertes Alkyl($C_1$-$C_8$) steht,

wobei

$R^7$ für gegebenenfalls durch Fluor, Chlor und/oder Brom substituiertes Alkyl($C_1$-$C_8$) steht,

$R^8$ und $R^9$ gleich oder verschieden sind und für Alkyl($C_1$-$C_8$) stehen oder gemeinsam mit dem Stickstoffatom, an das sie gebunden sind einen 3- bis 7-gliedrigen Ring bilden, der gegebenenfalls ein Sauerstoffatom enthält, für gegebenenfalls durch Fluor, Chlor, Brom oder Halogenalkyl($C_1$-$C_4$) substituiertes Cycloalkyl($C_3$-$C_6$), für jeweils gegebenenfalls durch Fluor, Chlor, Brom oder Alkoxy($C_1$-$C_4$) substituiertes Alkenyl($C_3$-$C_7$) oder Alkinyl($C_3$-$C_7$), für Formyl, für jeweils gegebenenfalls durch Fluor, Chlor und/oder Brom substituiertes Alkyl($C_1$-$C_8$)carbonyl oder Alkoxy($C_1$-$C_8$)carbonyl steht,

$R^6$ für gegebenenfalls durch Fluor, Chlor, Brom oder Halogenalkoxy($C_1$-$C_3$) substituiertes Alkyl($C_1$-$C_6$), für gegebenenfalls durch Fluor, Chlor, Brom, Halogenalkyl($C_1$-$C_3$) oder Halogenalkoxy($C_1$-$C_3$) substituiertes Cycloalkyl($C_3$-$C_6$) oder für den Rest

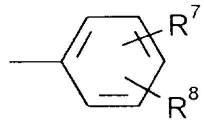

steht,

wobei $R^7$ und $R^8$ gleich oder verschieden sein können und für Wasserstoff, Fluor, Chlor, Brom, Iod. Alkyl($C_1$-$C_4$), Nitro, Cyano, Halogenalkyl($C_1$-$C_3$), Alkoxy($C_1$-$C_4$), Halogenalkoxy($C_1$-$C_3$) Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$) oder Alkyl($C_1$-$C_3$) substituiertes Phenoxy, gegebenenfalls durch Fluor, Chlor, Brom oder Alkoxy($C_1$-$C_3$) substituiertes Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)-thio substituiertes Cycloalkyl($C_3$-$C_6$) stehen oder wobei

$R^7$ und $R^8$ zusammen für einen der folgenden bivalenten Reste stehen:

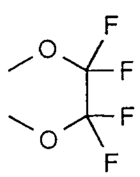
,
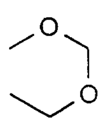
,
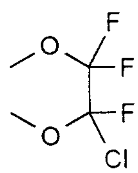
,
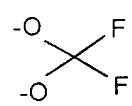
.

X für Sauerstoff oder Schwefel steht,

Y und Z unabhängig voneinander für Wasserstoff, Alkyl($C_1$-$C_4$), Fluor, Chlor, Brom, Iod, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl($C_1$-$C_3$), Alkoxy($C_1$-$C_3$) oder Halogenalkyl($C_1$-$C_3$) substituiertes Phenoxy oder Phenylthio, Halogenalkoxy($C_1$-$C_3$)carbonyl, Alkenyl($C_2$-$C_4$), Alkinyl($C_2$-$C_4$), Alkyl-($C_1$-$C_3$)thionyl, Alkyl($C_1$-$C_3$)sulfonyl, Halogenalkyl($C_1$-$C_3$)sulfonyl, gegebenenfalls durch Halogen, Alkoxy($C_1$-$C_3$) substituiertes Mono- oder Dialkyl($C_1$-$C_3$)amino, Nitro oder Cyano stehen oder

Y und Z gemeinsam für gegebenenfalls durch Fluor und/oder Chlor substituiertes 3,4-Methylendioxy oder 3,4-Ethylendioxy stehen.

4. Substituierte Pyrazolinderivate der Formel (I) gemäß Anspruch 1, in welcher

$R^1$ für einen gegebenenfalls ein- oder zweifach, gleich oder verschieden durch Chlor, Brom, Jod, Alkyl($C_1$-$C_3$), CN, $NO_2$, Alkoxy($C_1$-$C_3$)carbonyl, Alkyl($C_1$-$C_2$)thio, Alkoxy-($C_1$-$C_3$), Halogenalkyl($C_1$-$C_3$), Halogenalkyl($C_1$-$C_2$)thio, Dialkyl($C_1$-$C_2$)amino oder Halogenalkoxy($C_1$-$C_3$)carbonyl substituierten 1H-Pyrrolyl-(1)-, 1H-Pyrazolyl-(1)-, 1H-Imidazolyl-(1)-, 2H-1,2,3-Triazolyl-(2)-, 1H-1,2,3-Triazolyl-(1)-, 1H-1,2,4-Triazolyl-(1)-, 4H-1,2,4-Triazolyl-(4)-, 2H-Tetrazolyl-(2) oder 1H-Tetrazolyl-(1)-Rest steht,

$R^2$ für Wasserstoff, Alkyl($C_1$-$C_3$), Halogenalkyl($C_1$-$C_2$), Alkoxy($C_1$-$C_3$)carbonyl oder

Trialkyl($C_1$-$C_3$)silyl steht,

$R^3$ für Wasserstoff, Methyl, Ethyl oder Propyl steht,

$R^4$ für Wasserstoff, Methyl, Ethyl oder Propyl steht,

$R^5$ für Cyano, für gegebenenfalls durch Fluor, Chlor, Alkoxy($C_1$-$C_3$) oder einen der Reste $CO_2R^7$ oder $NR^8R^9$ substituiertes Alkyl($C_1$-$C_6$) steht,

wobei

$R^7$ für gegebenenfalls durch Fluor und/oder Chlor substituiertes Alkyl($C_1$-$C_6$) steht,

$R^8$ und $R^9$ gleich oder verschieden sind und für Alkyl($C_1$-$C_6$) stehen oder gemeinsam mit dem Stickstoffatom, an das sie gebunden sind einen 5- oder 6-gliedrigen Ring bilden, der gegebenenfalls ein Sauerstoffatom enthält,

für gegebenenfalls durch Fluor, Chlor oder Halogenalkyl($C_1$-$C_3$) substituiertes Cycloalkyl mit 3, 5 oder 6 C-Atomen, für jeweils gegebenenfalls durch Fluor, Chlor oder Alkoxy($C_1$-$C_3$) substituiertes Alkenyl($C_3$-$C_6$) oder Alkinyl($C_3$-$C_6$), für Formyl, für jeweils gegebenenfalls durch Fluor und/oder Chlor substituiertes Alkyl($C_1$-$C_6$)carbonyl oder Alkoxy($C_1$-$C_6$)carbonyl steht,

$R^6$ für gegebenenfalls durch Fluor, Chlor, Halogenalkyl($C_1$-$C_3$) oder Halogenalkoxy($C_1$-$C_2$) substituiertes Cycloalkyl($C_3$-$C_6$) oder für den Rest

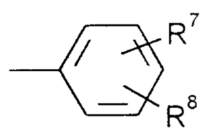

steht,

wobei $R^7$ und $R^8$ gleich oder verschieden sein können und für Wasserstoff, Fluor, Chlor, Brom, Alkyl($C_1$-$C_3$), Nitro, Cyano, Halogenalkyl($C_1$-$C_3$), Alkoxy($C_1$-$C_3$), Halogenalkoxy($C_1$-$C_2$), Alkyl($C_1$-$C_2$)thio, Halogenalkyl($C_1$-$C_2$)thio, gegebenenfalls durch Fluor, Chlor, Brom, Halogenalkyl($C_1$-$C_2$), Alkoxy($C_1$-$C_2$) oder Alkyl($C_1$-$C_2$) substituiertes Phenoxy, gegebenenfalls durch Fluor, Chlor oder Alkoxy($C_1$-$C_3$) substituiertes Mono- oder Dialkylamino mit jeweils 1 bis 3 Kohlenstoffatomen im Alkylrest, gegebenenfalls durch Alkyl($C_1$-$C_2$), Alkoxy($C_1$-$C_2$), Fluor, Chlor oder Alkyl($C_1$-$C_2$)thio substituiertes Cycloalkyl mit 3, 5 oder 6 C-Atomen stehen oder

$R^7$ und $R^8$ zusammen für eine der folgenden bivalenten Reste stehen:

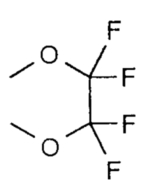 , 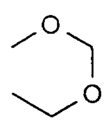 , 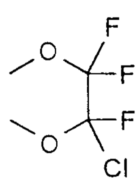 , 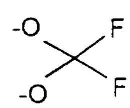 ,

X für Sauerstoff oder Schwefel stellt,

Y und Z unabhängig voneinander für Wasserstoff, Alkyl($C_1$-$C_3$), Fluor, Chlor, Brom, Halogenalkyl($C_1$-$C_3$), Alkoxy($C_1$-$C_3$), Alkyl($C_1$-$C_3$)thio, Halogenalkoxy($C_1$-$C_2$), Halogenalkyl($C_1$-$C_2$)thio, Alkoxy($C_1$-$C_2$)carbonyl, gegebenenfalls durch Fluor, Chlor, Brom, Alkyl($C_1$-$C_3$), Alkoxy($C_1$-$C_3$) oder Halogenalkyl($C_1$-$C_3$) substituiertes Phenoxy, Halogenalkoxy($C_1$-$C_2$)carbonyl, Alkenyl($C_2$-$C_4$), Alkyl($C_1$-$C_3$)thionyl, Alkyl($C_1$-$C_3$)-sulfonyl, Halogenalkyl($C_1$-$C_3$)sulfonyl, gegebenenfalls durch Fluor, Chlor, Alkoxy($C_1$-$C_2$), substituiertes Mono- oder Dialkyl($C_1$-$C_3$)amino, Nitro oder Cyano stehen oder

Y und Z gemeinsam für gegebenenfalls durch Fluor und/oder Chlor substituiertes 3,4-Methylendioxy oder 3,4-Ethylendioxy stehen.

**5.** Verfahren zur Herstellung der substituierten Pyrazolinderivate der Formel (I) gemäß Anspruch 1, dadurch gekennzeichnet, daß man

a) substituierte Pyrazolinderivate der Formel (II)

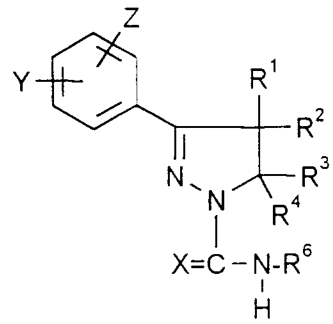

(II)

in welcher
$R^1$, $R^2$, $R^3$, $R^4$, $R^6$, X, Y und Z die in Anspruch 1 angegebene Bedeutung haben, mit Verbindungen der Formel (III)

$R^5$-A (III)

in welcher

$R^5$ die in Anspruch 1 angegebene Bedeutung hat und
A für eine Abgangsgruppe steht

in Gegenwart einer Base und gegebenenfalls in Gegenwart eines Verdünnungsmittels umsetzt oder
b) substituierte Pyrazoline der Formel (IV)

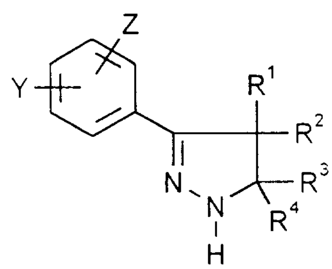

(IV)

in welcher
$R^1$, $R^2$, $R^3$, $R^4$, Y und Z die oben angegebene Bedeutung haben, mit Verbindungen der Formel (V)

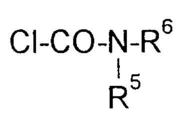

(V)

in welcher
$R^5$ und $R^6$ die oben angegebene Bedeutung haben
gegebenenfalls in Gegenwart einer Base und gegebenenfalls in Gegenwart eines Verdünnungsmittels umsetzt.

**6.** Schädlingsbekämpfungsmittel, gekennzeichnet durch einen Gehalt an mindestens einem substituierten Pyrazolinderivat der Formel (I) gemäß Anspruch 1.

**7.** Verwendung von substituierten Pyrazolinderivaten der Formel (I) gemäß Anspruch 1 zur Bekämpfung von Schädlingen.

**8.** Verfahren zur Bekämpfung von Schädlingen, dadurch gekennzeichnet, daß man substituierte Parazolinderivate der Formel (I) gemäß Anspruch 1 auf Schädlinge und/oder ihren Lebensraum einwirken läßt.

**9.** Verfahren zur Herstellung von Schädlingsbekämpfungsmitteln, dadurch gekennzeichnet, daß man substituierte Pyrazolinderivate der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung
EP 95 10 5723

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | EP-A-0 438 690 (BAYER AG)<br>* Ansprüche 1-3,7-10 * | 1-4,6-9 | C07D231/38<br>C07D403/04<br>A01N47/38 |
| X | EP-A-0 532 918 (BAYER AG)<br>* Ansprüche 1-5,8-11 * | 1-4,6-9 | |
| A | EP-A-0 515 893 (BAYER AG)<br>* Seite 19, vorletzte Verbindung in Tabelle 1 *<br>* Ansprüche 1-4,7-10 * | 1-4,6-9 | |
| A | EP-A-0 300 692 (E.I. DU PONT DE NEMOURS AND CO.)<br>* Seite 25 - Seite 26; Ansprüche 1,14,15 * | 1-4,6,8 | |
| A | EP-A-0 113 213 (FBC LTD.)<br>* Seite 20, Verbindungen 75 und 77; Seite 21, Verbindung 97 *<br>* Ansprüche 1,11,12 * | 1-4,6,8 | |
| A | WO-A-93 24464 (SCHERING AG)<br>* Seiten 7, 8, Beispiele 2, 7-10, 12 *<br>* Ansprüche 1,6-8 * | 1-4,6-8 | RECHERCHIERTE SACHGEBIETE (Int.Cl.6)<br>C07D<br>A01N |

Der vorliegende Recherchenbericht wurde für alle Patentansprüche erstellt

| Recherchenort | Abschlußdatum der Recherche | Prüfer |
|---|---|---|
| BERLIN | 31. Juli 1995 | Hass, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument